# EUROPEAN PATENT APPLICATION

(11) **EP 1 514 925 A1**
(43) Date of publication of application: **16.03.2005**
(21) Application number: 03730536.4
(22) Date of filing: 21.05.2003
(51) Int. Cl.: C12N 15/00, C07K 14/435, C07K 19/00, C12Q 1/02, C12N 1/15, C12N 1/19, C12N 1/21, C12N 5/00, A61K 45/00, A61P 25/28

(54) **METHOD OF SCREENING COMPOUND AFFECTING AMYLOID BETA PRODUCTION**

(30) Priority: 31.05.2002 JP 2002159472
(71) Applicant: OTSUKA PHARMACEUTICAL CO., LTD., Chiyoda-ku Tokyo 101-8535 (JP)
(72) Inventor: SHIMABUKU, Alfredo, Tokushima-shi, Tokushima 771-0112 (JP); OGINO, Koichi, Naruto-shi, Tokushima 772-0003 (JP); TAKI, Takao, Itano-gun, Tokushima 771-0205 (JP); SHIN, Ryong-Woon, Sendai-shi, Miyagi 989-3201 (JP); KITAMOTO, Tetsuyuki, Sendai-shi, Miyagi 980-0871 (JP)
(74) Representative: HOFFMANN EITLE
(86) International application number: PCT/JP2003/006319
(87) International publication number: WO 2003/102177

(57) **Abstract**

It is intended to provide a material for efficiently measuring the production of β-amyloid which relates to the onset of Alzheimer's type dementia, and a method of efficiently screening a substance affecting the production of β-amyloid by using the above material.

SEQUENCE LISTING

<110> OTSUKA PHARMACEUTICAL CO.,LTD.

<120> AMYLOIDOGENIC PROCESSING OF APP

<130> P03-34

<140>
<141>

<160> 21

<170> PatentIn Ver. 2.1

<210> 1
<211> 695
<212> PRT
<213> human

<400> 1
Met Leu Pro Gly Leu Ala Leu Leu Leu Leu Ala Ala Trp Thr Ala Arg
1               5                   10                  15

Ala Leu Glu Val Pro Thr Asp Gly Asn Ala Gly Leu Leu Ala Glu Pro
            20                  25                  30

Gln Ile Ala Met Phe Cys Gly Arg Leu Asn Met His Met Asn Val Gln
        35                  40                  45

Asn Gly Lys Trp Asp Ser Asp Pro Ser Gly Thr Lys Thr Cys Ile Asp
    50                  55                  60

Thr Lys Glu Gly Ile Leu Gln Tyr Cys Gln Glu Val Tyr Pro Glu Leu
65                  70                  75                  80

Gln Ile Thr Asn Val Val Glu Ala Asn Gln Pro Val Thr Ile Gln Asn
                85                  90                  95

Trp Cys Lys Arg Gly Arg Lys Gln Cys Lys Thr His Pro His Phe Val
            100                 105                 110

Ile Pro Tyr Arg Cys Leu Val Gly Glu Phe Val Ser Asp Ala Leu Leu
        115                 120                 125

Val Pro Asp Lys Cys Lys Phe Leu His Gln Glu Arg Met Asp Val Cys
    130                 135                 140

Glu Thr His Leu His Trp His Thr Val Ala Lys Glu Thr Cys Ser Glu
145                 150                 155                 160

Lys Ser Thr Asn Leu His Asp Tyr Gly Met Leu Leu Pro Cys Gly Ile
                165                 170                 175

Asp Lys Phe Arg Gly Val Glu Phe Val Cys Cys Pro Leu Ala Glu Glu
            180                 185                 190

Ser Asp Asn Val Asp Ser Ala Asp Ala Glu Glu Asp Asp Ser Asp Val
        195                 200                 205

Trp Trp Gly Gly Ala Asp Thr Asp Tyr Ala Asp Gly Ser Glu Asp Lys
    210                 215                 220

Val Val Glu Val Ala Glu Glu Glu Glu Val Ala Glu Val Glu Glu Glu
225                 230                 235                 240

Glu Ala Asp Asp Asp Glu Asp Asp Glu Asp Gly Asp Glu Val Glu Glu
                245                 250                 255

Glu Ala Glu Glu Pro Tyr Glu Glu Ala Thr Glu Arg Thr Thr Ser Ile
            260                 265                 270

Ala Thr Thr Thr Thr Thr Thr Thr Glu Ser Val Glu Glu Val Val Arg
        275                 280                 285

Val Pro Thr Thr Ala Ala Ser Thr Pro Asp Ala Val Asp Lys Tyr Leu
    290                 295                 300

Glu Thr Pro Gly Asp Glu Asn Glu His Ala His Phe Gln Lys Ala Lys
305                 310                 315                 320

Glu Arg Leu Glu Ala Lys His Arg Glu Arg Met Ser Gln Val Met Arg
                325                 330                 335

Glu Trp Glu Glu Ala Glu Arg Gln Ala Lys Asn Leu Pro Lys Ala Asp
            340                 345                 350

Lys Lys Ala Val Ile Gln His Phe Gln Glu Lys Val Glu Ser Leu Glu
        355                 360                 365

Gln Glu Ala Ala Asn Glu Arg Gln Gln Leu Val Glu Thr His Met Ala
    370                 375                 380

Arg Val Glu Ala Met Leu Asn Asp Arg Arg Arg Leu Ala Leu Glu Asn
385                 390                 395                 400

Tyr Ile Thr Ala Leu Gln Ala Val Pro Pro Arg Pro Arg His Val Phe
                405                 410                 415

Asn Met Leu Lys Lys Tyr Val Arg Ala Glu Gln Lys Asp Arg Gln His
            420                 425                 430

Thr Leu Lys His Phe Glu His Val Arg Met Val Asp Pro Lys Lys Ala
        435                 440                 445

Ala Gln Ile Arg Ser Gln Val Met Thr His Leu Arg Val Ile Tyr Glu
    450                 455                 460

Arg Met Asn Gln Ser Leu Ser Leu Leu Tyr Asn Val Pro Ala Val Ala
465                 470                 475                 480

Glu Glu Ile Gln Asp Glu Val Asp Glu Leu Leu Gln Lys Glu Gln Asn
                485                 490                 495

Tyr Ser Asp Asp Val Leu Ala Asn Met Ile Ser Glu Pro Arg Ile Ser
            500                 505                 510

Tyr Gly Asn Asp Ala Leu Met Pro Ser Leu Thr Glu Thr Lys Thr Thr
        515                 520                 525

Val Glu Leu Leu Pro Val Asn Gly Glu Phe Ser Leu Asp Asp Leu Gln
    530                 535                 540

Pro Trp His Ser Phe Gly Ala Asp Ser Val Pro Ala Asn Thr Glu Asn
545                 550                 555                 560

Glu Val Glu Pro Val Asp Ala Arg Pro Ala Ala Asp Arg Gly Leu Thr
                565                 570                 575

Thr Arg Pro Gly Ser Gly Leu Thr Asn Ile Lys Thr Glu Glu Ile Ser
            580                 585                 590

Glu Val Lys Met Asp Ala Glu Phe Arg His Asp Ser Gly Tyr Glu Val
        595                 600                 605

His His Gln Lys Leu Val Phe Phe Ala Glu Asp Val Gly Ser Asn Lys
    610                 615                 620

Gly Ala Ile Ile Gly Leu Met Val Gly Gly Val Val Ile Ala Thr Val
625                 630                 635                 640

Ile Val Ile Thr Leu Val Met Leu Lys Lys Lys Gln Tyr Thr Ser Ile
                645                 650                 655

His His Gly Val Val Glu Val Asp Ala Ala Val Thr Pro Glu Glu Arg
            660                 665                 670

His Leu Ser Lys Met Gln Gln Asn Gly Tyr Glu Asn Pro Thr Tyr Lys
        675                 680                 685

Phe Phe Glu Gln Met Gln Asn
    690                 695

<210> 2
<211> 42
<212> PRT
<213> Artificial Sequence

<220>
<223> Description of Artificial Sequence:peptide

<400> 2
Lys Met Asp Ala Glu Phe Arg His Asp Ser Gly Tyr Glu Val His His
1               5                   10                  15

Gln Trp Leu Val Phe Phe Ala Glu Asp Val Gly Ser Asn Lys Gly Ala
            20                  25                  30

Ile Ile Gly Leu Met Val Gly Gly Val Val
        35                  40

<210> 3
<211> 42
<212> PRT
<213> Artificial Sequence

<220>
<223> Description of Artificial Sequence:peptide

<400> 3
Lys Met Asp Ala Glu Phe Arg His Asp Ser Gly Tyr Glu Val His His
 1               5                   10                  15

Gln Lys Ala Val Phe Phe Ala Glu Asp Val Gly Ser Asn Lys Gly Ala
            20                  25                  30

Ile Ile Gly Leu Met Val Gly Gly Val Val
        35                  40

<210> 4
<211> 42
<212> PRT
<213> Artificial Sequence

<220>
<223> Description of Artificial Sequence:peptide

<400> 4
Lys Met Asp Ala Glu Phe Arg His Asp Ser Gly Tyr Glu Val His His
 1               5                   10                  15

Gln Lys Glu Val Phe Phe Ala Glu Asp Val Gly Ser Asn Lys Gly Ala
            20                  25                  30

Ile Ile Gly Leu Met Val Gly Gly Val Val
        35                  40

<210> 5
<211> 42
<212> PRT
<213> Artificial Sequence

<220>
<223> Description of Artificial Sequence:peptide

<400> 5
Lys Met Asp Ala Glu Phe Arg His Asp Ser Gly Tyr Glu Val His His
1               5                   10                  15

Gln Lys Lys Val Phe Phe Ala Glu Asp Val Gly Ser Asn Lys Gly Ala
            20                  25                  30

Ile Ile Gly Leu Met Val Gly Gly Val Val
        35                  40

<210> 6
<211> 42
<212> PRT
<213> Artificial Sequence

<220>
<223> Description of Artificial Sequence:peptide

<400> 6
Lys Met Asp Ala Glu Phe Arg His Asp Ser Gly Tyr Glu Val His His
1               5                   10                  15

Gln Lys Pro Val Phe Phe Ala Glu Asp Val Gly Ser Asn Lys Gly Ala
            20                  25                  30

Ile Ile Gly Leu Met Val Gly Gly Val Val
        35                  40

<210> 7
<211> 42
<212> PRT

<213> Artificial Sequence

<220>
<223> Description of Artificial Sequence:peptide

<400> 7
Asn Leu Asp Ala Glu Phe Arg His Asp Ser Gly Tyr Glu Val His His
 1               5                   10                  15

Gln Trp Leu Val Phe Phe Ala Glu Asp Val Gly Ser Asn Lys Gly Ala
            20                  25                  30

Ile Ile Gly Leu Met Val Gly Gly Val Val
        35                  40

<210> 8
<211> 42
<212> PRT
<213> Artificial Sequence

<220>
<223> Description of Artificial Sequence:peptide

<400> 8
Asn Leu Asp Ala Glu Phe Arg His Asp Ser Gly Tyr Glu Val His His
 1               5                   10                  15

Gln Lys Ala Val Phe Phe Ala Glu Asp Val Gly Ser Asn Lys Gly Ala
            20                  25                  30

Ile Ile Gly Leu Met Val Gly Gly Val Val
        35                  40

<210> 9

<211> 42
<212> PRT
<213> Artificial Sequence

<220>
<223> Description of Artificial Sequence:peptide

<400> 9
Asn Leu Asp Ala Glu Phe Arg His Asp Ser Gly Tyr Glu Val His His
 1               5                   10                  15

Gln Lys Glu Val Phe Phe Ala Glu Asp Val Gly Ser Asn Lys Gly Ala
            20                  25                  30

Ile Ile Gly Leu Met Val Gly Gly Val Val
        35                  40


<210> 10
<211> 42
<212> PRT
<213> Artificial Sequence

<220>
<223> Description of Artificial Sequence:peptide

<400> 10
Asn Leu Asp Ala Glu Phe Arg His Asp Ser Gly Tyr Glu Val His His
 1               5                   10                  15

Gln Lys Lys Val Phe Phe Ala Glu Asp Val Gly Ser Asn Lys Gly Ala
            20                  25                  30

Ile Ile Gly Leu Met Val Gly Gly Val Val
        35                  40

<210> 11
<211> 42
<212> PRT
<213> Artificial Sequence

<220>
<223> Description of Artificial Sequence:peptide

<400> 11
Asn Leu Asp Ala Glu Phe Arg His Asp Ser Gly Tyr Glu Val His His
1               5                   10                  15

Gln Lys Pro Val Phe Phe Ala Glu Asp Val Gly Ser Asn Lys Gly Ala
            20                  25                  30

Ile Ile Gly Leu Met Val Gly Gly Val Val
        35                  40


<210> 12
<211> 626
<212> PRT
<213> Artificial Sequence

<220>
<223> Description of Artificial Sequence:fusion

<400> 12
Met Leu Leu Leu Leu Leu Leu Leu Gly Leu Arg Leu Gln Leu Ser Leu
1               5                   10                  15

Gly Ile Ile Pro Val Glu Glu Glu Asn Pro Asp Phe Trp Asn Arg Glu
            20                  25                  30

Ala Ala Glu Ala Leu Gly Ala Ala Lys Lys Leu Gln Pro Ala Gln Thr

        35                  40                  45

Ala Ala Lys Asn Leu Ile Ile Phe Leu Gly Asp Gly Met Gly Val Ser
    50                  55                  60

Thr Val Thr Ala Ala Arg Ile Leu Lys Gly Gln Lys Lys Asp Lys Leu
65                  70                  75                  80

Gly Pro Glu Ile Pro Leu Ala Met Asp Arg Phe Pro Tyr Val Ala Leu
                85                  90                  95

Ser Lys Thr Tyr Asn Val Asp Lys His Val Pro Asp Ser Gly Ala Thr
            100                 105                 110

Ala Thr Ala Tyr Leu Cys Gly Val Lys Gly Asn Phe Gln Thr Ile Gly
        115                 120                 125

Leu Ser Ala Ala Ala Arg Phe Asn Gln Cys Asn Thr Thr Arg Gly Asn
    130                 135                 140

Glu Val Ile Ser Val Met Asn Arg Ala Lys Lys Ala Gly Lys Ser Val
145                 150                 155                 160

Gly Val Val Thr Thr Thr Arg Val Gln His Ala Ser Pro Ala Gly Thr
                165                 170                 175

Tyr Ala His Thr Val Asn Arg Asn Trp Tyr Ser Asp Ala Asp Val Pro
            180                 185                 190

Ala Ser Ala Arg Gln Glu Gly Cys Gln Asp Ile Ala Thr Gln Leu Ile
        195                 200                 205

Ser Asn Met Asp Ile Asp Val Ile Leu Gly Gly Gly Arg Lys Tyr Met
    210                 215                 220

Phe Arg Met Gly Thr Pro Asp Pro Glu Tyr Pro Asp Asp Tyr Ser Gln

225                 230                 235                 240

Gly Gly Thr Arg Leu Asp Gly Lys Asn Leu Val Gln Glu Trp Leu Ala
                245                 250                 255

Lys Arg Gln Gly Ala Arg Tyr Val Trp Asn Arg Thr Glu Leu Met Gln
            260                 265                 270

Ala Ser Leu Asp Pro Ser Val Thr His Leu Met Gly Leu Phe Glu Pro
        275                 280                 285

Gly Asp Met Lys Tyr Glu Ile His Arg Asp Ser Thr Leu Asp Pro Ser
    290                 295                 300

Leu Met Glu Met Thr Glu Ala Ala Leu Arg Leu Leu Ser Arg Asn Pro
305                 310                 315                 320

Arg Gly Phe Phe Leu Phe Val Glu Gly Gly Arg Ile Asp His Gly His
                325                 330                 335

His Glu Ser Arg Ala Tyr Arg Ala Leu Thr Glu Thr Ile Met Phe Asp
            340                 345                 350

Asp Ala Ile Glu Arg Ala Gly Gln Leu Thr Ser Glu Glu Asp Thr Leu
        355                 360                 365

Ser Leu Val Thr Ala Asp His Ser His Val Phe Ser Phe Gly Gly Tyr
    370                 375                 380

Pro Leu Arg Gly Ser Ser Ile Phe Gly Leu Ala Pro Gly Lys Ala Arg
385                 390                 395                 400

Asp Arg Lys Ala Tyr Thr Val Leu Leu Tyr Gly Asn Gly Pro Gly Tyr
                405                 410                 415

Val Leu Lys Asp Gly Ala Arg Pro Asp Val Thr Glu Ser Glu Ser Gly

            420                 425                 430

Ser Pro Glu Tyr Arg Gln Gln Ser Ala Val Pro Leu Asp Glu Glu Thr
        435                 440                 445

His Ala Gly Glu Asp Val Ala Val Phe Ala Arg Gly Pro Gln Ala His
    450                 455                 460

Leu Val His Gly Val Gln Glu Gln Thr Phe Ile Ala His Val Met Ala
465                 470                 475                 480

Phe Ala Ala Cys Leu Glu Pro Tyr Thr Ala Cys Asp Leu Ala Pro Pro
                485                 490                 495

Ala Gly Thr Thr Asp Ala Ala His Pro Gly Tyr Ser Arg Pro Gly Ser
            500                 505                 510

Gly Leu Thr Asn Ile Lys Thr Glu Glu Ile Ser Glu Val Lys Met Asp
        515                 520                 525

Ala Glu Phe Arg His Asp Ser Gly Tyr Glu Val His His Gln Trp Leu
    530                 535                 540

Val Phe Phe Ala Glu Asp Val Gly Ser Asn Lys Gly Ala Ile Ile Gly
545                 550                 555                 560

Leu Met Val Gly Gly Val Val Ile Ala Thr Val Ile Val Ile Thr Leu
                565                 570                 575

Val Met Leu Lys Lys Lys Gln Tyr Thr Ser Ile His His Gly Val Val
            580                 585                 590

Glu Val Asp Ala Ala Val Thr Pro Glu Glu Arg His Leu Ser Lys Met
        595                 600                 605

Gln Gln Asn Gly Tyr Glu Asn Pro Thr Tyr Lys Phe Phe Glu Gln Met

610                 615                 620

Gln Asn
625

<210> 13
<211> 626
<212> PRT
<213> Artificial Sequence

<220>
<223> Description of Artificial Sequence:fusion

<400> 13
Met Leu Leu Leu Leu Leu Leu Leu Gly Leu Arg Leu Gln Leu Ser Leu
  1               5                  10                  15

Gly Ile Ile Pro Val Glu Glu Glu Asn Pro Asp Phe Trp Asn Arg Glu
             20                  25                  30

Ala Ala Glu Ala Leu Gly Ala Ala Lys Lys Leu Gln Pro Ala Gln Thr
         35                  40                  45

Ala Ala Lys Asn Leu Ile Ile Phe Leu Gly Asp Gly Met Gly Val Ser
     50                  55                  60

Thr Val Thr Ala Ala Arg Ile Leu Lys Gly Gln Lys Lys Asp Lys Leu
 65                  70                  75                  80

Gly Pro Glu Ile Pro Leu Ala Met Asp Arg Phe Pro Tyr Val Ala Leu
                 85                  90                  95

Ser Lys Thr Tyr Asn Val Asp Lys His Val Pro Asp Ser Gly Ala Thr
            100                 105                 110

Ala Thr Ala Tyr Leu Cys Gly Val Lys Gly Asn Phe Gln Thr Ile Gly
        115                 120                 125

Leu Ser Ala Ala Ala Arg Phe Asn Gln Cys Asn Thr Thr Arg Gly Asn
    130                 135                 140

Glu Val Ile Ser Val Met Asn Arg Ala Lys Lys Ala Gly Lys Ser Val
145                 150                 155                 160

Gly Val Val Thr Thr Thr Arg Val Gln His Ala Ser Pro Ala Gly Thr
                165                 170                 175

Tyr Ala His Thr Val Asn Arg Asn Trp Tyr Ser Asp Ala Asp Val Pro
            180                 185                 190

Ala Ser Ala Arg Gln Glu Gly Cys Gln Asp Ile Ala Thr Gln Leu Ile
        195                 200                 205

Ser Asn Met Asp Ile Asp Val Ile Leu Gly Gly Gly Arg Lys Tyr Met
    210                 215                 220

Phe Arg Met Gly Thr Pro Asp Pro Glu Tyr Pro Asp Asp Tyr Ser Gln
225                 230                 235                 240

Gly Gly Thr Arg Leu Asp Gly Lys Asn Leu Val Gln Glu Trp Leu Ala
                245                 250                 255

Lys Arg Gln Gly Ala Arg Tyr Val Trp Asn Arg Thr Glu Leu Met Gln
            260                 265                 270

Ala Ser Leu Asp Pro Ser Val Thr His Leu Met Gly Leu Phe Glu Pro
        275                 280                 285

Gly Asp Met Lys Tyr Glu Ile His Arg Asp Ser Thr Leu Asp Pro Ser
    290                 295                 300

Leu Met Glu Met Thr Glu Ala Ala Leu Arg Leu Leu Ser Arg Asn Pro
305                 310                 315                 320

Arg Gly Phe Phe Leu Phe Val Glu Gly Gly Arg Ile Asp His Gly His
                325                 330                 335

His Glu Ser Arg Ala Tyr Arg Ala Leu Thr Glu Thr Ile Met Phe Asp
            340                 345                 350

Asp Ala Ile Glu Arg Ala Gly Gln Leu Thr Ser Glu Glu Asp Thr Leu
        355                 360                 365

Ser Leu Val Thr Ala Asp His Ser His Val Phe Ser Phe Gly Gly Tyr
    370                 375                 380

Pro Leu Arg Gly Ser Ser Ile Phe Gly Leu Ala Pro Gly Lys Ala Arg
385                 390                 395                 400

Asp Arg Lys Ala Tyr Thr Val Leu Leu Tyr Gly Asn Gly Pro Gly Tyr
                405                 410                 415

Val Leu Lys Asp Gly Ala Arg Pro Asp Val Thr Glu Ser Glu Ser Gly
            420                 425                 430

Ser Pro Glu Tyr Arg Gln Gln Ser Ala Val Pro Leu Asp Glu Glu Thr
        435                 440                 445

His Ala Gly Glu Asp Val Ala Val Phe Ala Arg Gly Pro Gln Ala His
    450                 455                 460

Leu Val His Gly Val Gln Glu Gln Thr Phe Ile Ala His Val Met Ala
465                 470                 475                 480

Phe Ala Ala Cys Leu Glu Pro Tyr Thr Ala Cys Asp Leu Ala Pro Pro
                485                 490                 495

Ala Gly Thr Thr Asp Ala Ala His Pro Gly Tyr Ser Arg Pro Gly Ser
            500                 505                 510

Gly Leu Thr Asn Ile Lys Thr Glu Glu Ile Ser Glu Val Lys Met Asp
        515                 520                 525

Ala Glu Phe Arg His Asp Ser Gly Tyr Glu Val His His Gln Lys Ala
    530                 535                 540

Val Phe Phe Ala Glu Asp Val Gly Ser Asn Lys Gly Ala Ile Ile Gly
545                 550                 555                 560

Leu Met Val Gly Gly Val Val Ile Ala Thr Val Ile Val Ile Thr Leu
                565                 570                 575

Val Met Leu Lys Lys Lys Gln Tyr Thr Ser Ile His His Gly Val Val
            580                 585                 590

Glu Val Asp Ala Ala Val Thr Pro Glu Glu Arg His Leu Ser Lys Met
        595                 600                 605

Gln Gln Asn Gly Tyr Glu Asn Pro Thr Tyr Lys Phe Phe Glu Gln Met
    610                 615                 620

Gln Asn
625

<210> 14
<211> 626
<212> PRT
<213> Artificial Sequence

<220>
<223> Description of Artificial Sequence:fusion

<400> 14
Met Leu Leu Leu Leu Leu Leu Leu Gly Leu Arg Leu Gln Leu Ser Leu
1               5                   10                  15

Gly Ile Ile Pro Val Glu Glu Glu Asn Pro Asp Phe Trp Asn Arg Glu
            20                  25                  30

Ala Ala Glu Ala Leu Gly Ala Ala Lys Lys Leu Gln Pro Ala Gln Thr
        35                  40                  45

Ala Ala Lys Asn Leu Ile Ile Phe Leu Gly Asp Gly Met Gly Val Ser
    50                  55                  60

Thr Val Thr Ala Ala Arg Ile Leu Lys Gly Gln Lys Lys Asp Lys Leu
65                  70                  75                  80

Gly Pro Glu Ile Pro Leu Ala Met Asp Arg Phe Pro Tyr Val Ala Leu
                85                  90                  95

Ser Lys Thr Tyr Asn Val Asp Lys His Val Pro Asp Ser Gly Ala Thr
            100                 105                 110

Ala Thr Ala Tyr Leu Cys Gly Val Lys Gly Asn Phe Gln Thr Ile Gly
        115                 120                 125

Leu Ser Ala Ala Ala Arg Phe Asn Gln Cys Asn Thr Thr Arg Gly Asn
    130                 135                 140

Glu Val Ile Ser Val Met Asn Arg Ala Lys Lys Ala Gly Lys Ser Val
145                 150                 155                 160

Gly Val Val Thr Thr Thr Arg Val Gln His Ala Ser Pro Ala Gly Thr
                165                 170                 175

Tyr Ala His Thr Val Asn Arg Asn Trp Tyr Ser Asp Ala Asp Val Pro
            180                 185                 190

Ala Ser Ala Arg Gln Glu Gly Cys Gln Asp Ile Ala Thr Gln Leu Ile
        195                 200                 205
Ser Asn Met Asp Ile Asp Val Ile Leu Gly Gly Gly Arg Lys Tyr Met
    210                 215                 220
Phe Arg Met Gly Thr Pro Asp Pro Glu Tyr Pro Asp Asp Tyr Ser Gln
225                 230                 235                 240
Gly Gly Thr Arg Leu Asp Gly Lys Asn Leu Val Gln Glu Trp Leu Ala
                245                 250                 255
Lys Arg Gln Gly Ala Arg Tyr Val Trp Asn Arg Thr Glu Leu Met Gln
            260                 265                 270
Ala Ser Leu Asp Pro Ser Val Thr His Leu Met Gly Leu Phe Glu Pro
        275                 280                 285
Gly Asp Met Lys Tyr Glu Ile His Arg Asp Ser Thr Leu Asp Pro Ser
    290                 295                 300
Leu Met Glu Met Thr Glu Ala Ala Leu Arg Leu Leu Ser Arg Asn Pro
305                 310                 315                 320
Arg Gly Phe Phe Leu Phe Val Glu Gly Gly Arg Ile Asp His Gly His
                325                 330                 335
His Glu Ser Arg Ala Tyr Arg Ala Leu Thr Glu Thr Ile Met Phe Asp
            340                 345                 350
Asp Ala Ile Glu Arg Ala Gly Gln Leu Thr Ser Glu Glu Asp Thr Leu
        355                 360                 365
Ser Leu Val Thr Ala Asp His Ser His Val Phe Ser Phe Gly Gly Tyr
    370                 375                 380

Pro Leu Arg Gly Ser Ser Ile Phe Gly Leu Ala Pro Gly Lys Ala Arg
385                 390                 395                 400

Asp Arg Lys Ala Tyr Thr Val Leu Leu Tyr Gly Asn Gly Pro Gly Tyr
                405                 410                 415

Val Leu Lys Asp Gly Ala Arg Pro Asp Val Thr Glu Ser Glu Ser Gly
            420                 425                 430

Ser Pro Glu Tyr Arg Gln Gln Ser Ala Val Pro Leu Asp Glu Glu Thr
        435                 440                 445

His Ala Gly Glu Asp Val Ala Val Phe Ala Arg Gly Pro Gln Ala His
    450                 455                 460

Leu Val His Gly Val Gln Glu Gln Thr Phe Ile Ala His Val Met Ala
465                 470                 475                 480

Phe Ala Ala Cys Leu Glu Pro Tyr Thr Ala Cys Asp Leu Ala Pro Pro
                485                 490                 495

Ala Gly Thr Thr Asp Ala Ala His Pro Gly Tyr Ser Arg Pro Gly Ser
            500                 505                 510

Gly Leu Thr Asn Ile Lys Thr Glu Glu Ile Ser Glu Val Lys Met Asp
        515                 520                 525

Ala Glu Phe Arg His Asp Ser Gly Tyr Glu Val His His Gln Lys Glu
    530                 535                 540

Val Phe Phe Ala Glu Asp Val Gly Ser Asn Lys Gly Ala Ile Ile Gly
545                 550                 555                 560

Leu Met Val Gly Gly Val Val Ile Ala Thr Val Ile Val Ile Thr Leu
                565                 570                 575

Val Met Leu Lys Lys Lys Gln Tyr Thr Ser Ile His His Gly Val Val
            580                 585                 590

Glu Val Asp Ala Ala Val Thr Pro Glu Glu Arg His Leu Ser Lys Met
        595                 600                 605

Gln Gln Asn Gly Tyr Glu Asn Pro Thr Tyr Lys Phe Phe Glu Gln Met
    610                 615                 620

Gln Asn
625

<210> 15
<211> 626
<212> PRT
<213> Artificial Sequence

<220>
<223> Description of Artificial Sequence:fusion

<400> 15
Met Leu Leu Leu Leu Leu Leu Leu Gly Leu Arg Leu Gln Leu Ser Leu
1               5                   10                  15

Gly Ile Ile Pro Val Glu Glu Glu Asn Pro Asp Phe Trp Asn Arg Glu
            20                  25                  30

Ala Ala Glu Ala Leu Gly Ala Ala Lys Lys Leu Gln Pro Ala Gln Thr
        35                  40                  45

Ala Ala Lys Asn Leu Ile Ile Phe Leu Gly Asp Gly Met Gly Val Ser
    50                  55                  60

Thr Val Thr Ala Ala Arg Ile Leu Lys Gly Gln Lys Lys Asp Lys Leu

65                  70                  75                  80

Gly Pro Glu Ile Pro Leu Ala Met Asp Arg Phe Pro Tyr Val Ala Leu
                85                  90                  95

Ser Lys Thr Tyr Asn Val Asp Lys His Val Pro Asp Ser Gly Ala Thr
            100                 105                 110

Ala Thr Ala Tyr Leu Cys Gly Val Lys Gly Asn Phe Gln Thr Ile Gly
        115                 120                 125

Leu Ser Ala Ala Ala Arg Phe Asn Gln Cys Asn Thr Thr Arg Gly Asn
    130                 135                 140

Glu Val Ile Ser Val Met Asn Arg Ala Lys Lys Ala Gly Lys Ser Val
145                 150                 155                 160

Gly Val Val Thr Thr Thr Arg Val Gln His Ala Ser Pro Ala Gly Thr
                165                 170                 175

Tyr Ala His Thr Val Asn Arg Asn Trp Tyr Ser Asp Ala Asp Val Pro
            180                 185                 190

Ala Ser Ala Arg Gln Glu Gly Cys Gln Asp Ile Ala Thr Gln Leu Ile
        195                 200                 205

Ser Asn Met Asp Ile Asp Val Ile Leu Gly Gly Gly Arg Lys Tyr Met
    210                 215                 220

Phe Arg Met Gly Thr Pro Asp Pro Glu Tyr Pro Asp Asp Tyr Ser Gln
225                 230                 235                 240

Gly Gly Thr Arg Leu Asp Gly Lys Asn Leu Val Gln Glu Trp Leu Ala
                245                 250                 255

Lys Arg Gln Gly Ala Arg Tyr Val Trp Asn Arg Thr Glu Leu Met Gln

            260                 265                 270

Ala Ser Leu Asp Pro Ser Val Thr His Leu Met Gly Leu Phe Glu Pro
        275                 280                 285

Gly Asp Met Lys Tyr Glu Ile His Arg Asp Ser Thr Leu Asp Pro Ser
    290                 295                 300

Leu Met Glu Met Thr Glu Ala Ala Leu Arg Leu Leu Ser Arg Asn Pro
305                 310                 315                 320

Arg Gly Phe Phe Leu Phe Val Glu Gly Gly Arg Ile Asp His Gly His
                325                 330                 335

His Glu Ser Arg Ala Tyr Arg Ala Leu Thr Glu Thr Ile Met Phe Asp
            340                 345                 350

Asp Ala Ile Glu Arg Ala Gly Gln Leu Thr Ser Glu Glu Asp Thr Leu
        355                 360                 365

Ser Leu Val Thr Ala Asp His Ser His Val Phe Ser Phe Gly Gly Tyr
    370                 375                 380

Pro Leu Arg Gly Ser Ser Ile Phe Gly Leu Ala Pro Gly Lys Ala Arg
385                 390                 395                 400

Asp Arg Lys Ala Tyr Thr Val Leu Leu Tyr Gly Asn Gly Pro Gly Tyr
                405                 410                 415

Val Leu Lys Asp Gly Ala Arg Pro Asp Val Thr Glu Ser Glu Ser Gly
            420                 425                 430

Ser Pro Glu Tyr Arg Gln Gln Ser Ala Val Pro Leu Asp Glu Glu Thr
        435                 440                 445

His Ala Gly Glu Asp Val Ala Val Phe Ala Arg Gly Pro Gln Ala His

450                 455                 460

Leu Val His Gly Val Gln Glu Gln Thr Phe Ile Ala His Val Met Ala
465                 470                 475                 480

Phe Ala Ala Cys Leu Glu Pro Tyr Thr Ala Cys Asp Leu Ala Pro Pro
                485                 490                 495

Ala Gly Thr Thr Asp Ala Ala His Pro Gly Tyr Ser Arg Pro Gly Ser
            500                 505                 510

Gly Leu Thr Asn Ile Lys Thr Glu Glu Ile Ser Glu Val Lys Met Asp
        515                 520                 525

Ala Glu Phe Arg His Asp Ser Gly Tyr Glu Val His His Gln Lys Lys
    530                 535                 540

Val Phe Phe Ala Glu Asp Val Gly Ser Asn Lys Gly Ala Ile Ile Gly
545                 550                 555                 560

Leu Met Val Gly Gly Val Val Ile Ala Thr Val Ile Val Ile Thr Leu
                565                 570                 575

Val Met Leu Lys Lys Lys Gln Tyr Thr Ser Ile His His Gly Val Val
            580                 585                 590

Glu Val Asp Ala Ala Val Thr Pro Glu Glu Arg His Leu Ser Lys Met
        595                 600                 605

Gln Gln Asn Gly Tyr Glu Asn Pro Thr Tyr Lys Phe Phe Glu Gln Met
    610                 615                 620

Gln Asn
625

<210> 16
<211> 626
<212> PRT
<213> Artificial Sequence

<220>
<223> Description of Artificial Sequence:fusion

<400> 16
Met Leu Leu Leu Leu Leu Leu Leu Gly Leu Arg Leu Gln Leu Ser Leu
1               5                   10                  15

Gly Ile Ile Pro Val Glu Glu Glu Asn Pro Asp Phe Trp Asn Arg Glu
            20                  25                  30

Ala Ala Glu Ala Leu Gly Ala Ala Lys Lys Leu Gln Pro Ala Gln Thr
        35                  40                  45

Ala Ala Lys Asn Leu Ile Ile Phe Leu Gly Asp Gly Met Gly Val Ser
    50                  55                  60

Thr Val Thr Ala Ala Arg Ile Leu Lys Gly Gln Lys Lys Asp Lys Leu
65                  70                  75                  80

Gly Pro Glu Ile Pro Leu Ala Met Asp Arg Phe Pro Tyr Val Ala Leu
                85                  90                  95

Ser Lys Thr Tyr Asn Val Asp Lys His Val Pro Asp Ser Gly Ala Thr
            100                 105                 110

Ala Thr Ala Tyr Leu Cys Gly Val Lys Gly Asn Phe Gln Thr Ile Gly
        115                 120                 125

Leu Ser Ala Ala Ala Arg Phe Asn Gln Cys Asn Thr Thr Arg Gly Asn
    130                 135                 140

Glu Val Ile Ser Val Met Asn Arg Ala Lys Lys Ala Gly Lys Ser Val
145                 150                 155                 160

Gly Val Val Thr Thr Thr Arg Val Gln His Ala Ser Pro Ala Gly Thr
                165                 170                 175

Tyr Ala His Thr Val Asn Arg Asn Trp Tyr Ser Asp Ala Asp Val Pro
            180                 185                 190

Ala Ser Ala Arg Gln Glu Gly Cys Gln Asp Ile Ala Thr Gln Leu Ile
        195                 200                 205

Ser Asn Met Asp Ile Asp Val Ile Leu Gly Gly Gly Arg Lys Tyr Met
    210                 215                 220

Phe Arg Met Gly Thr Pro Asp Pro Glu Tyr Pro Asp Asp Tyr Ser Gln
225                 230                 235                 240

Gly Gly Thr Arg Leu Asp Gly Lys Asn Leu Val Gln Glu Trp Leu Ala
                245                 250                 255

Lys Arg Gln Gly Ala Arg Tyr Val Trp Asn Arg Thr Glu Leu Met Gln
            260                 265                 270

Ala Ser Leu Asp Pro Ser Val Thr His Leu Met Gly Leu Phe Glu Pro
        275                 280                 285

Gly Asp Met Lys Tyr Glu Ile His Arg Asp Ser Thr Leu Asp Pro Ser
    290                 295                 300

Leu Met Glu Met Thr Glu Ala Ala Leu Arg Leu Leu Ser Arg Asn Pro
305                 310                 315                 320

Arg Gly Phe Phe Leu Phe Val Glu Gly Gly Arg Ile Asp His Gly His
                325                 330                 335

His Glu Ser Arg Ala Tyr Arg Ala Leu Thr Glu Thr Ile Met Phe Asp
            340                 345                 350

Asp Ala Ile Glu Arg Ala Gly Gln Leu Thr Ser Glu Glu Asp Thr Leu
        355                 360                 365

Ser Leu Val Thr Ala Asp His Ser His Val Phe Ser Phe Gly Gly Tyr
    370                 375                 380

Pro Leu Arg Gly Ser Ser Ile Phe Gly Leu Ala Pro Gly Lys Ala Arg
385                 390                 395                 400

Asp Arg Lys Ala Tyr Thr Val Leu Leu Tyr Gly Asn Gly Pro Gly Tyr
                405                 410                 415

Val Leu Lys Asp Gly Ala Arg Pro Asp Val Thr Glu Ser Glu Ser Gly
            420                 425                 430

Ser Pro Glu Tyr Arg Gln Gln Ser Ala Val Pro Leu Asp Glu Glu Thr
        435                 440                 445

His Ala Gly Glu Asp Val Ala Val Phe Ala Arg Gly Pro Gln Ala His
    450                 455                 460

Leu Val His Gly Val Gln Glu Gln Thr Phe Ile Ala His Val Met Ala
465                 470                 475                 480

Phe Ala Ala Cys Leu Glu Pro Tyr Thr Ala Cys Asp Leu Ala Pro Pro
                485                 490                 495

Ala Gly Thr Thr Asp Ala Ala His Pro Gly Tyr Ser Arg Pro Gly Ser
            500                 505                 510

Gly Leu Thr Asn Ile Lys Thr Glu Glu Ile Ser Glu Val Lys Met Asp
        515                 520                 525

Ala Glu Phe Arg His Asp Ser Gly Tyr Glu Val His His Gln Lys Pro
    530                 535                 540

Val Phe Phe Ala Glu Asp Val Gly Ser Asn Lys Gly Ala Ile Ile Gly
545                 550                 555                 560

Leu Met Val Gly Gly Val Val Ile Ala Thr Val Ile Val Ile Thr Leu
                565                 570                 575

Val Met Leu Lys Lys Lys Gln Tyr Thr Ser Ile His His Gly Val Val
            580                 585                 590

Glu Val Asp Ala Ala Val Thr Pro Glu Glu Arg His Leu Ser Lys Met
        595                 600                 605

Gln Gln Asn Gly Tyr Glu Asn Pro Thr Tyr Lys Phe Phe Glu Gln Met
    610                 615                 620

Gln Asn
625

<210> 17
<211> 626
<212> PRT
<213> Artificial Sequence

<220>
<223> Description of Artificial Sequence:fusion

<400> 17
Met Leu Leu Leu Leu Leu Leu Leu Gly Leu Arg Leu Gln Leu Ser Leu
1               5                   10                  15

Gly Ile Ile Pro Val Glu Glu Glu Asn Pro Asp Phe Trp Asn Arg Glu
            20                  25                  30

Ala Ala Glu Ala Leu Gly Ala Ala Lys Lys Leu Gln Pro Ala Gln Thr
        35                  40                  45
Ala Ala Lys Asn Leu Ile Ile Phe Leu Gly Asp Gly Met Gly Val Ser
    50                  55                  60
Thr Val Thr Ala Ala Arg Ile Leu Lys Gly Gln Lys Lys Asp Lys Leu
65                  70                  75                  80
Gly Pro Glu Ile Pro Leu Ala Met Asp Arg Phe Pro Tyr Val Ala Leu
                85                  90                  95
Ser Lys Thr Tyr Asn Val Asp Lys His Val Pro Asp Ser Gly Ala Thr
            100                 105                 110
Ala Thr Ala Tyr Leu Cys Gly Val Lys Gly Asn Phe Gln Thr Ile Gly
        115                 120                 125
Leu Ser Ala Ala Ala Arg Phe Asn Gln Cys Asn Thr Thr Arg Gly Asn
    130                 135                 140
Glu Val Ile Ser Val Met Asn Arg Ala Lys Lys Ala Gly Lys Ser Val
145                 150                 155                 160
Gly Val Val Thr Thr Thr Arg Val Gln His Ala Ser Pro Ala Gly Thr
                165                 170                 175
Tyr Ala His Thr Val Asn Arg Asn Trp Tyr Ser Asp Ala Asp Val Pro
            180                 185                 190
Ala Ser Ala Arg Gln Glu Gly Cys Gln Asp Ile Ala Thr Gln Leu Ile
        195                 200                 205
Ser Asn Met Asp Ile Asp Val Ile Leu Gly Gly Gly Arg Lys Tyr Met
    210                 215                 220

Phe Arg Met Gly Thr Pro Asp Pro Glu Tyr Pro Asp Asp Tyr Ser Gln
225                 230                 235                 240

Gly Gly Thr Arg Leu Asp Gly Lys Asn Leu Val Gln Glu Trp Leu Ala
                245                 250                 255

Lys Arg Gln Gly Ala Arg Tyr Val Trp Asn Arg Thr Glu Leu Met Gln
            260                 265                 270

Ala Ser Leu Asp Pro Ser Val Thr His Leu Met Gly Leu Phe Glu Pro
        275                 280                 285

Gly Asp Met Lys Tyr Glu Ile His Arg Asp Ser Thr Leu Asp Pro Ser
    290                 295                 300

Leu Met Glu Met Thr Glu Ala Ala Leu Arg Leu Leu Ser Arg Asn Pro
305                 310                 315                 320

Arg Gly Phe Phe Leu Phe Val Glu Gly Gly Arg Ile Asp His Gly His
                325                 330                 335

His Glu Ser Arg Ala Tyr Arg Ala Leu Thr Glu Thr Ile Met Phe Asp
            340                 345                 350

Asp Ala Ile Glu Arg Ala Gly Gln Leu Thr Ser Glu Glu Asp Thr Leu
        355                 360                 365

Ser Leu Val Thr Ala Asp His Ser His Val Phe Ser Phe Gly Gly Tyr
    370                 375                 380

Pro Leu Arg Gly Ser Ser Ile Phe Gly Leu Ala Pro Gly Lys Ala Arg
385                 390                 395                 400

Asp Arg Lys Ala Tyr Thr Val Leu Leu Tyr Gly Asn Gly Pro Gly Tyr
                405                 410                 415

Val Leu Lys Asp Gly Ala Arg Pro Asp Val Thr Glu Ser Glu Ser Gly
            420                 425                 430

Ser Pro Glu Tyr Arg Gln Gln Ser Ala Val Pro Leu Asp Glu Glu Thr
        435                 440                 445

His Ala Gly Glu Asp Val Ala Val Phe Ala Arg Gly Pro Gln Ala His
    450                 455                 460

Leu Val His Gly Val Gln Glu Gln Thr Phe Ile Ala His Val Met Ala
465                 470                 475                 480

Phe Ala Ala Cys Leu Glu Pro Tyr Thr Ala Cys Asp Leu Ala Pro Pro
                485                 490                 495

Ala Gly Thr Thr Asp Ala Ala His Pro Gly Tyr Ser Arg Pro Gly Ser
            500                 505                 510

Gly Leu Thr Asn Ile Lys Thr Glu Glu Ile Ser Glu Val Asn Leu Asp
        515                 520                 525

Ala Glu Phe Arg His Asp Ser Gly Tyr Glu Val His His Gln Trp Leu
    530                 535                 540

Val Phe Phe Ala Glu Asp Val Gly Ser Asn Lys Gly Ala Ile Ile Gly
545                 550                 555                 560

Leu Met Val Gly Gly Val Val Ile Ala Thr Val Ile Val Ile Thr Leu
                565                 570                 575

Val Met Leu Lys Lys Lys Gln Tyr Thr Ser Ile His His Gly Val Val
            580                 585                 590

Glu Val Asp Ala Ala Val Thr Pro Glu Glu Arg His Leu Ser Lys Met
        595                 600                 605

Gln Gln Asn Gly Tyr Glu Asn Pro Thr Tyr Lys Phe Phe Glu Gln Met
    610                 615                 620

Gln Asn
625

<210> 18
<211> 626
<212> PRT
<213> Artificial Sequence

<220>
<223> Description of Artificial Sequence:fusion

<400> 18
Met Leu Leu Leu Leu Leu Leu Leu Gly Leu Arg Leu Gln Leu Ser Leu
  1               5                  10                  15

Gly Ile Ile Pro Val Glu Glu Glu Asn Pro Asp Phe Trp Asn Arg Glu
             20                  25                  30

Ala Ala Glu Ala Leu Gly Ala Ala Lys Lys Leu Gln Pro Ala Gln Thr
         35                  40                  45

Ala Ala Lys Asn Leu Ile Ile Phe Leu Gly Asp Gly Met Gly Val Ser
     50                  55                  60

Thr Val Thr Ala Ala Arg Ile Leu Lys Gly Gln Lys Lys Asp Lys Leu
 65                  70                  75                  80

Gly Pro Glu Ile Pro Leu Ala Met Asp Arg Phe Pro Tyr Val Ala Leu
                 85                  90                  95

Ser Lys Thr Tyr Asn Val Asp Lys His Val Pro Asp Ser Gly Ala Thr

            100                 105                 110

Ala Thr Ala Tyr Leu Cys Gly Val Lys Gly Asn Phe Gln Thr Ile Gly
        115                 120                 125

Leu Ser Ala Ala Ala Arg Phe Asn Gln Cys Asn Thr Thr Arg Gly Asn
    130                 135                 140

Glu Val Ile Ser Val Met Asn Arg Ala Lys Lys Ala Gly Lys Ser Val
145                 150                 155                 160

Gly Val Val Thr Thr Thr Arg Val Gln His Ala Ser Pro Ala Gly Thr
                165                 170                 175

Tyr Ala His Thr Val Asn Arg Asn Trp Tyr Ser Asp Ala Asp Val Pro
            180                 185                 190

Ala Ser Ala Arg Gln Glu Gly Cys Gln Asp Ile Ala Thr Gln Leu Ile
        195                 200                 205

Ser Asn Met Asp Ile Asp Val Ile Leu Gly Gly Gly Arg Lys Tyr Met
    210                 215                 220

Phe Arg Met Gly Thr Pro Asp Pro Glu Tyr Pro Asp Asp Tyr Ser Gln
225                 230                 235                 240

Gly Gly Thr Arg Leu Asp Gly Lys Asn Leu Val Gln Glu Trp Leu Ala
                245                 250                 255

Lys Arg Gln Gly Ala Arg Tyr Val Trp Asn Arg Thr Glu Leu Met Gln
            260                 265                 270

Ala Ser Leu Asp Pro Ser Val Thr His Leu Met Gly Leu Phe Glu Pro
        275                 280                 285

Gly Asp Met Lys Tyr Glu Ile His Arg Asp Ser Thr Leu Asp Pro Ser

290                 295                 300

Leu Met Glu Met Thr Glu Ala Ala Leu Arg Leu Leu Ser Arg Asn Pro
305                 310                 315                 320

Arg Gly Phe Phe Leu Phe Val Glu Gly Gly Arg Ile Asp His Gly His
                325                 330                 335

His Glu Ser Arg Ala Tyr Arg Ala Leu Thr Glu Thr Ile Met Phe Asp
            340                 345                 350

Asp Ala Ile Glu Arg Ala Gly Gln Leu Thr Ser Glu Glu Asp Thr Leu
        355                 360                 365

Ser Leu Val Thr Ala Asp His Ser His Val Phe Ser Phe Gly Gly Tyr
    370                 375                 380

Pro Leu Arg Gly Ser Ser Ile Phe Gly Leu Ala Pro Gly Lys Ala Arg
385                 390                 395                 400

Asp Arg Lys Ala Tyr Thr Val Leu Leu Tyr Gly Asn Gly Pro Gly Tyr
                405                 410                 415

Val Leu Lys Asp Gly Ala Arg Pro Asp Val Thr Glu Ser Glu Ser Gly
            420                 425                 430

Ser Pro Glu Tyr Arg Gln Gln Ser Ala Val Pro Leu Asp Glu Glu Thr
        435                 440                 445

His Ala Gly Glu Asp Val Ala Val Phe Ala Arg Gly Pro Gln Ala His
    450                 455                 460

Leu Val His Gly Val Gln Glu Gln Thr Phe Ile Ala His Val Met Ala
465                 470                 475                 480

Phe Ala Ala Cys Leu Glu Pro Tyr Thr Ala Cys Asp Leu Ala Pro Pro

                485                 490                 495

Ala Gly Thr Thr Asp Ala Ala His Pro Gly Tyr Ser Arg Pro Gly Ser
            500                 505                 510

Gly Leu Thr Asn Ile Lys Thr Glu Glu Ile Ser Glu Val Asn Leu Asp
        515                 520                 525

Ala Glu Phe Arg His Asp Ser Gly Tyr Glu Val His His Gln Lys Ala
    530                 535                 540

Val Phe Phe Ala Glu Asp Val Gly Ser Asn Lys Gly Ala Ile Ile Gly
545                 550                 555                 560

Leu Met Val Gly Gly Val Val Ile Ala Thr Val Ile Val Ile Thr Leu
                565                 570                 575

Val Met Leu Lys Lys Lys Gln Tyr Thr Ser Ile His His Gly Val Val
            580                 585                 590

Glu Val Asp Ala Ala Val Thr Pro Glu Glu Arg His Leu Ser Lys Met
        595                 600                 605

Gln Gln Asn Gly Tyr Glu Asn Pro Thr Tyr Lys Phe Phe Glu Gln Met
    610                 615                 620

Gln Asn
625

<210> 19
<211> 626
<212> PRT
<213> Artificial Sequence

<220>

<223> Description of Artificial Sequence:fusion

<400> 19
Met Leu Leu Leu Leu Leu Leu Leu Gly Leu Arg Leu Gln Leu Ser Leu
1               5                   10                  15

Gly Ile Ile Pro Val Glu Glu Glu Asn Pro Asp Phe Trp Asn Arg Glu
            20                  25                  30

Ala Ala Glu Ala Leu Gly Ala Ala Lys Lys Leu Gln Pro Ala Gln Thr
        35                  40                  45

Ala Ala Lys Asn Leu Ile Ile Phe Leu Gly Asp Gly Met Gly Val Ser
    50                  55                  60

Thr Val Thr Ala Ala Arg Ile Leu Lys Gly Gln Lys Lys Asp Lys Leu
65                  70                  75                  80

Gly Pro Glu Ile Pro Leu Ala Met Asp Arg Phe Pro Tyr Val Ala Leu
                85                  90                  95

Ser Lys Thr Tyr Asn Val Asp Lys His Val Pro Asp Ser Gly Ala Thr
            100                 105                 110

Ala Thr Ala Tyr Leu Cys Gly Val Lys Gly Asn Phe Gln Thr Ile Gly
        115                 120                 125

Leu Ser Ala Ala Ala Arg Phe Asn Gln Cys Asn Thr Thr Arg Gly Asn
    130                 135                 140

Glu Val Ile Ser Val Met Asn Arg Ala Lys Lys Ala Gly Lys Ser Val
145                 150                 155                 160

Gly Val Val Thr Thr Thr Arg Val Gln His Ala Ser Pro Ala Gly Thr
                165                 170                 175

Tyr Ala His Thr Val Asn Arg Asn Trp Tyr Ser Asp Ala Asp Val Pro
            180                 185                 190

Ala Ser Ala Arg Gln Glu Gly Cys Gln Asp Ile Ala Thr Gln Leu Ile
        195                 200                 205

Ser Asn Met Asp Ile Asp Val Ile Leu Gly Gly Gly Arg Lys Tyr Met
    210                 215                 220

Phe Arg Met Gly Thr Pro Asp Pro Glu Tyr Pro Asp Asp Tyr Ser Gln
225                 230                 235                 240

Gly Gly Thr Arg Leu Asp Gly Lys Asn Leu Val Gln Glu Trp Leu Ala
                245                 250                 255

Lys Arg Gln Gly Ala Arg Tyr Val Trp Asn Arg Thr Glu Leu Met Gln
            260                 265                 270

Ala Ser Leu Asp Pro Ser Val Thr His Leu Met Gly Leu Phe Glu Pro
        275                 280                 285

Gly Asp Met Lys Tyr Glu Ile His Arg Asp Ser Thr Leu Asp Pro Ser
    290                 295                 300

Leu Met Glu Met Thr Glu Ala Ala Leu Arg Leu Leu Ser Arg Asn Pro
305                 310                 315                 320

Arg Gly Phe Phe Leu Phe Val Glu Gly Gly Arg Ile Asp His Gly His
                325                 330                 335

His Glu Ser Arg Ala Tyr Arg Ala Leu Thr Glu Thr Ile Met Phe Asp
            340                 345                 350

Asp Ala Ile Glu Arg Ala Gly Gln Leu Thr Ser Glu Glu Asp Thr Leu
        355                 360                 365

Ser Leu Val Thr Ala Asp His Ser His Val Phe Ser Phe Gly Gly Tyr
    370                 375                 380

Pro Leu Arg Gly Ser Ser Ile Phe Gly Leu Ala Pro Gly Lys Ala Arg
385                 390                 395                 400

Asp Arg Lys Ala Tyr Thr Val Leu Leu Tyr Gly Asn Gly Pro Gly Tyr
                405                 410                 415

Val Leu Lys Asp Gly Ala Arg Pro Asp Val Thr Glu Ser Glu Ser Gly
            420                 425                 430

Ser Pro Glu Tyr Arg Gln Gln Ser Ala Val Pro Leu Asp Glu Glu Thr
        435                 440                 445

His Ala Gly Glu Asp Val Ala Val Phe Ala Arg Gly Pro Gln Ala His
    450                 455                 460

Leu Val His Gly Val Gln Glu Gln Thr Phe Ile Ala His Val Met Ala
465                 470                 475                 480

Phe Ala Ala Cys Leu Glu Pro Tyr Thr Ala Cys Asp Leu Ala Pro Pro
                485                 490                 495

Ala Gly Thr Thr Asp Ala Ala His Pro Gly Tyr Ser Arg Pro Gly Ser
            500                 505                 510

Gly Leu Thr Asn Ile Lys Thr Glu Glu Ile Ser Glu Val Asn Leu Asp
        515                 520                 525

Ala Glu Phe Arg His Asp Ser Gly Tyr Glu Val His His Gln Lys Glu
    530                 535                 540

Val Phe Phe Ala Glu Asp Val Gly Ser Asn Lys Gly Ala Ile Ile Gly
545                 550                 555                 560

Leu Met Val Gly Gly Val Val Ile Ala Thr Val Ile Val Ile Thr Leu
                565                 570                 575

Val Met Leu Lys Lys Lys Gln Tyr Thr Ser Ile His His Gly Val Val
            580                 585                 590

Glu Val Asp Ala Ala Val Thr Pro Glu Glu Arg His Leu Ser Lys Met
        595                 600                 605

Gln Gln Asn Gly Tyr Glu Asn Pro Thr Tyr Lys Phe Phe Glu Gln Met
    610                 615                 620

Gln Asn
625

<210> 20
<211> 626
<212> PRT
<213> Artificial Sequence

<220>
<223> Description of Artificial Sequence:fusion

<400> 20
Met Leu Leu Leu Leu Leu Leu Leu Gly Leu Arg Leu Gln Leu Ser Leu
  1               5                  10                  15

Gly Ile Ile Pro Val Glu Glu Glu Asn Pro Asp Phe Trp Asn Arg Glu
             20                  25                  30

Ala Ala Glu Ala Leu Gly Ala Ala Lys Lys Leu Gln Pro Ala Gln Thr
         35                  40                  45

Ala Ala Lys Asn Leu Ile Ile Phe Leu Gly Asp Gly Met Gly Val Ser
     50                  55                  60

Thr Val Thr Ala Ala Arg Ile Leu Lys Gly Gln Lys Lys Asp Lys Leu
65                  70                  75                  80

Gly Pro Glu Ile Pro Leu Ala Met Asp Arg Phe Pro Tyr Val Ala Leu
                85                  90                  95

Ser Lys Thr Tyr Asn Val Asp Lys His Val Pro Asp Ser Gly Ala Thr
            100                 105                 110

Ala Thr Ala Tyr Leu Cys Gly Val Lys Gly Asn Phe Gln Thr Ile Gly
        115                 120                 125

Leu Ser Ala Ala Ala Arg Phe Asn Gln Cys Asn Thr Thr Arg Gly Asn
    130                 135                 140

Glu Val Ile Ser Val Met Asn Arg Ala Lys Lys Ala Gly Lys Ser Val
145                 150                 155                 160

Gly Val Val Thr Thr Thr Arg Val Gln His Ala Ser Pro Ala Gly Thr
                165                 170                 175

Tyr Ala His Thr Val Asn Arg Asn Trp Tyr Ser Asp Ala Asp Val Pro
            180                 185                 190

Ala Ser Ala Arg Gln Glu Gly Cys Gln Asp Ile Ala Thr Gln Leu Ile
        195                 200                 205

Ser Asn Met Asp Ile Asp Val Ile Leu Gly Gly Gly Arg Lys Tyr Met
    210                 215                 220

Phe Arg Met Gly Thr Pro Asp Pro Glu Tyr Pro Asp Asp Tyr Ser Gln
225                 230                 235                 240

Gly Gly Thr Arg Leu Asp Gly Lys Asn Leu Val Gln Glu Trp Leu Ala
                245                 250                 255

Lys Arg Gln Gly Ala Arg Tyr Val Trp Asn Arg Thr Glu Leu Met Gln
            260                 265                 270

Ala Ser Leu Asp Pro Ser Val Thr His Leu Met Gly Leu Phe Glu Pro
        275                 280                 285

Gly Asp Met Lys Tyr Glu Ile His Arg Asp Ser Thr Leu Asp Pro Ser
    290                 295                 300

Leu Met Glu Met Thr Glu Ala Ala Leu Arg Leu Leu Ser Arg Asn Pro
305                 310                 315                 320

Arg Gly Phe Phe Leu Phe Val Glu Gly Gly Arg Ile Asp His Gly His
                325                 330                 335

His Glu Ser Arg Ala Tyr Arg Ala Leu Thr Glu Thr Ile Met Phe Asp
            340                 345                 350

Asp Ala Ile Glu Arg Ala Gly Gln Leu Thr Ser Glu Glu Asp Thr Leu
        355                 360                 365

Ser Leu Val Thr Ala Asp His Ser His Val Phe Ser Phe Gly Gly Tyr
    370                 375                 380

Pro Leu Arg Gly Ser Ser Ile Phe Gly Leu Ala Pro Gly Lys Ala Arg
385                 390                 395                 400

Asp Arg Lys Ala Tyr Thr Val Leu Leu Tyr Gly Asn Gly Pro Gly Tyr
                405                 410                 415

Val Leu Lys Asp Gly Ala Arg Pro Asp Val Thr Glu Ser Glu Ser Gly
            420                 425                 430

Ser Pro Glu Tyr Arg Gln Gln Ser Ala Val Pro Leu Asp Glu Glu Thr
        435                 440                 445

His Ala Gly Glu Asp Val Ala Val Phe Ala Arg Gly Pro Gln Ala His
    450                 455                 460

Leu Val His Gly Val Gln Glu Gln Thr Phe Ile Ala His Val Met Ala
465                 470                 475                 480

Phe Ala Ala Cys Leu Glu Pro Tyr Thr Ala Cys Asp Leu Ala Pro Pro
                485                 490                 495

Ala Gly Thr Thr Asp Ala Ala His Pro Gly Tyr Ser Arg Pro Gly Ser
            500                 505                 510

Gly Leu Thr Asn Ile Lys Thr Glu Glu Ile Ser Glu Val Asn Leu Asp
        515                 520                 525

Ala Glu Phe Arg His Asp Ser Gly Tyr Glu Val His His Gln Lys Lys
    530                 535                 540

Val Phe Phe Ala Glu Asp Val Gly Ser Asn Lys Gly Ala Ile Ile Gly
545                 550                 555                 560

Leu Met Val Gly Gly Val Val Ile Ala Thr Val Ile Val Ile Thr Leu
                565                 570                 575

Val Met Leu Lys Lys Lys Gln Tyr Thr Ser Ile His His Gly Val Val
            580                 585                 590

Glu Val Asp Ala Ala Val Thr Pro Glu Glu Arg His Leu Ser Lys Met
        595                 600                 605

Gln Gln Asn Gly Tyr Glu Asn Pro Thr Tyr Lys Phe Phe Glu Gln Met
    610                 615                 620

Gln Asn
625

<210> 21
<211> 626
<212> PRT
<213> Artificial Sequence

<220>
<223> Description of Artificial Sequence:fusion

<400> 21
Met Leu Leu Leu Leu Leu Leu Leu Gly Leu Arg Leu Gln Leu Ser Leu
1               5                   10                  15

Gly Ile Ile Pro Val Glu Glu Glu Asn Pro Asp Phe Trp Asn Arg Glu
            20                  25                  30

Ala Ala Glu Ala Leu Gly Ala Ala Lys Lys Leu Gln Pro Ala Gln Thr
        35                  40                  45

Ala Ala Lys Asn Leu Ile Ile Phe Leu Gly Asp Gly Met Gly Val Ser
    50                  55                  60

Thr Val Thr Ala Ala Arg Ile Leu Lys Gly Gln Lys Lys Asp Lys Leu
65                  70                  75                  80

Gly Pro Glu Ile Pro Leu Ala Met Asp Arg Phe Pro Tyr Val Ala Leu
                85                  90                  95

Ser Lys Thr Tyr Asn Val Asp Lys His Val Pro Asp Ser Gly Ala Thr
            100                 105                 110

Ala Thr Ala Tyr Leu Cys Gly Val Lys Gly Asn Phe Gln Thr Ile Gly
        115                 120                 125

Leu Ser Ala Ala Ala Arg Phe Asn Gln Cys Asn Thr Thr Arg Gly Asn

    130                 135                 140

Glu Val Ile Ser Val Met Asn Arg Ala Lys Lys Ala Gly Lys Ser Val
145                 150                 155                 160

Gly Val Val Thr Thr Thr Arg Val Gln His Ala Ser Pro Ala Gly Thr
                165                 170                 175

Tyr Ala His Thr Val Asn Arg Asn Trp Tyr Ser Asp Ala Asp Val Pro
            180                 185                 190

Ala Ser Ala Arg Gln Glu Gly Cys Gln Asp Ile Ala Thr Gln Leu Ile
        195                 200                 205

Ser Asn Met Asp Ile Asp Val Ile Leu Gly Gly Gly Arg Lys Tyr Met
    210                 215                 220

Phe Arg Met Gly Thr Pro Asp Pro Glu Tyr Pro Asp Asp Tyr Ser Gln
225                 230                 235                 240

Gly Gly Thr Arg Leu Asp Gly Lys Asn Leu Val Gln Glu Trp Leu Ala
                245                 250                 255

Lys Arg Gln Gly Ala Arg Tyr Val Trp Asn Arg Thr Glu Leu Met Gln
            260                 265                 270

Ala Ser Leu Asp Pro Ser Val Thr His Leu Met Gly Leu Phe Glu Pro
        275                 280                 285

Gly Asp Met Lys Tyr Glu Ile His Arg Asp Ser Thr Leu Asp Pro Ser
    290                 295                 300

Leu Met Glu Met Thr Glu Ala Ala Leu Arg Leu Leu Ser Arg Asn Pro
305                 310                 315                 320

Arg Gly Phe Phe Leu Phe Val Glu Gly Gly Arg Ile Asp His Gly His

                325                 330                 335
His Glu Ser Arg Ala Tyr Arg Ala Leu Thr Glu Thr Ile Met Phe Asp
            340                 345                 350
Asp Ala Ile Glu Arg Ala Gly Gln Leu Thr Ser Glu Glu Asp Thr Leu
        355                 360                 365
Ser Leu Val Thr Ala Asp His Ser His Val Phe Ser Phe Gly Gly Tyr
    370                 375                 380
Pro Leu Arg Gly Ser Ser Ile Phe Gly Leu Ala Pro Gly Lys Ala Arg
385                 390                 395                 400
Asp Arg Lys Ala Tyr Thr Val Leu Leu Tyr Gly Asn Gly Pro Gly Tyr
                405                 410                 415
Val Leu Lys Asp Gly Ala Arg Pro Asp Val Thr Glu Ser Glu Ser Gly
            420                 425                 430
Ser Pro Glu Tyr Arg Gln Gln Ser Ala Val Pro Leu Asp Glu Glu Thr
        435                 440                 445
His Ala Gly Glu Asp Val Ala Val Phe Ala Arg Gly Pro Gln Ala His
    450                 455                 460
Leu Val His Gly Val Gln Glu Gln Thr Phe Ile Ala His Val Met Ala
465                 470                 475                 480
Phe Ala Ala Cys Leu Glu Pro Tyr Thr Ala Cys Asp Leu Ala Pro Pro
                485                 490                 495
Ala Gly Thr Thr Asp Ala Ala His Pro Gly Tyr Ser Arg Pro Gly Ser
            500                 505                 510
Gly Leu Thr Asn Ile Lys Thr Glu Glu Ile Ser Glu Val Asn Leu Asp

        515                 520                 525

Ala Glu Phe Arg His Asp Ser Gly Tyr Glu Val His His Gln Lys Pro
    530                 535                 540

Val Phe Phe Ala Glu Asp Val Gly Ser Asn Lys Gly Ala Ile Ile Gly
545                 550                 555                 560

Leu Met Val Gly Gly Val Val Ile Ala Thr Val Ile Val Ile Thr Leu
                565                 570                 575

Val Met Leu Lys Lys Lys Gln Tyr Thr Ser Ile His His Gly Val Val
            580                 585                 590

Glu Val Asp Ala Ala Val Thr Pro Glu Glu Arg His Leu Ser Lys Met
        595                 600                 605

Gln Gln Asn Gly Tyr Glu Asn Pro Thr Tyr Lys Phe Phe Glu Gln Met
    610                 615                 620

Gln Asn
625

## Description

### TECHNICAL FIELD

The present invention relates to a method for measuring β-amyloid, which is considered to be one of the causes of Alzheimer's disease. The invention also relates to a method for screening a substance which affects the production of β-amyloid.

### BACKGROUND OF THE INVENTION

Alzheimer's-type dementia is a progressive neurodegenerative disease, and is characterized by cognitive dysfunction typified by recent-memory disturbance and pathological changes (a senile plaque, neurofibrillary degeneration, and neurocyte death).

β-amyloid is a polypeptide composed of about 40-amino acid residues, and is accepted as a principal component of the senile plaque that is a pathological feature of Alzheimer' s-type dementia. As currently known, accumulation of β-amyloid in the brain is observed as a pathological change in the early stages of this disease.

β-amyloid is considered to be one of the causes for the onset of this disorder for the following reasons: β-amyloid itself is toxic to neurocytes, there exists a family with genetic mutation in a precursor of a β-amyloid, i.e., aβ-amyloid precursor protein (APP) and the family develops Alzheimer's-type dementia with high frequency, and, in a transgenic mouse to which the mutant gene was introduced, a large amount of β-amyloid was accumulated in the brain.

APP is classified into a type I membrane protein by structure. In the living body, there are various kinds of molecular species distinguished by the existence or absence of the Kunitz-Type protease inhibitor domain. Although APP695, which is composed of 695 amino acid residues, is a principal molecule in the nervous system, the physiological function of APP 695 is almost unknown.

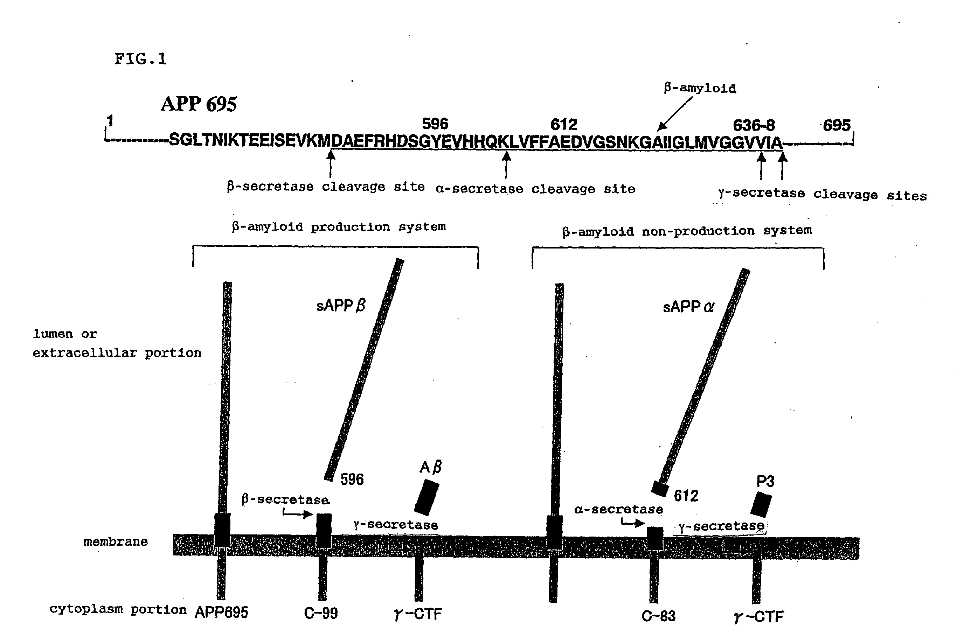

As shown in Fig. 1, most APPs are subjected to α-secretase processing at the β-amyloid central site (the 16th amino acid residue from N-terminus), i.e., at C terminal side of a lysine residue at position 612 of APP 695. In this case, the N-terminal sAPP-α and the C-terminal fragment (C83) composed of 83 residues are generated and β-amyloids are not produced. Further, a P3 peptide is generated from C83 by γ-secretase. However, the P3 peptide differs from β-Amyloid in its properties, and thus it is incohesive and easily-degradable (β-amyloid non-production system).

As a minor processing system for APP, there exists an amyloidgenic system which produces β-amyloid. This processing is a system in which β-secretase acts on APP 695 at the C terminal side of a methionine residue at position 596 of APP695 to generate sAPPβ and a C-terminal fragment of 99 residues (C99). C99 is further cleaved by γ-secretase to generate β-amyloid, which is considered to be a cause of Alzheimer's disease. This pathway may be 10% or less of the entire processing of APP (β-amyloid production system).

The α-secretase used herein denotes an enzyme which acts on the α-secretase cleavage site in APP to cleave a peptide bond.

In the early stage of the above-described APP processing, α- and β-secretases act in a competitive manner (Journal of Molecular Neuroscience 17, 157-180, 2001).

At present, some attempts are being made to prevent and/or treat Alzheimer's disease by inhibiting β-amyloid production, and β-secretase is being studied as one of the targeted candidate molecules. β-secretase has already been identified as BACE1. It is reported that mice deficient in BACE1 have a normal phenotype based on knowledge obtained from BACE1 knockout mice (Nature Neurosci., 4, 231, 2001 and Nature Neurosci., 4, 233, 2001). Therefore, the suppression of β-amyloid production by inhibiting β-secretase activity is expected to be effective in a prophylactic and/or therapeutic agent for Alzheimer's disease. A substance for improving β-secretase activity is useful for elucidating the onset mechanism of Alzheimer's disease, the production of a disease model, and proof of an amyloid hypothesis.

A method for screening a compound which has β-secretase inhibition activity is practically examined. The method comprises expressing in a cell a protein created by fusing the C terminus of a wild type APP into the C terminus of the secretory form of alkaline phosphatase (SEAP), and determining SEAP activity secreted in the culture supernatant (CIS Diagnostic Co., Ltd.).

However, since α- and β- secretases act in a competitive manner as described above, and α-secretase processing makes up 90% of all processing, β-amyloid, production efficiency is extremely low. As is clear from the above, it is unlikely that the screening system using the fusion protein will be able to efficiently screen substances which affect the β-amyloid production.

### DISCLOSURE OF THE INVENTION

A primary object of the invention is to provide a material and a process for efficiently measuring the production of β-Amyloid related to the onset of Alzheimer-type dementia. Another primary object of the invention is to provide a process for efficiently screening substances which affect the production of β-Amyloid.

The inventors conducted intensive research on a material and a process for efficiently measuring the production of β-amyloid and found that a specific amino acid substitution in APP eliminates the α-secretase cleavage without affecting β-secretase cleavage. The inventors also found that a substance which affects β-amyloid production can be efficiently screened by fusing the APP-modified protein into a detectable secretory protein and stably expressing the same in a cell. The inventors conducted further research and accomplished the present invention based on these findings.

The present invention includes the following items:
Item 1. A modified protein of β-amyloid precursor protein (APP), wherein (i) a β-secretase cleavage site is contained and (ii) one or more amino acids are mutated so that the β-secretase cleavage is not affected and α-secretase cleavage does not occur.
Item 2. A nucleic acid molecule which encodes an APP modified protein according to Item 1.
Item 3. A test cell which expresses an APP modified protein according to Item 1.
Item 4. A fusion protein, comprising an APP modified protein according to Item 1 and a detectable secretory protein.
Item 5. A nucleic acid molecule encoding a fusion protein, comprising an APP modified protein according to Item 1 and a detectable secretory protein.
Item 6. A test cell expressing a fusion protein comprising an APP modified protein according to Item 1 and a detectable secretory protein.
Item 7. A method for examining whether or not a subject substance affects β-secretase activity, the method comprising culturing in the presence of the subject substance a test cell expressing a fusion protein comprising an APP modified protein according to Item 1 and a detectable secretory protein, and measuring detectable secretory proteins which were cleaved by β-secretase and secreted outside the cell.
Item 8. A method for examining whether or not a subject substance affects β-amyloid production, the method comprising culturing in the presence of the subject substance a test cell expressing a fusion protein comprising an APP modified protein according to Item 1 and a detectable secretory protein, and measuring detectable secretory proteins which were cleaved by β-secretase and secreted outside the cell.
Item 9. A method for screening a substance useful as an active ingredient for a therapeutic agent or prophylactic agent for Alzheimer's-type dementia, the screening method comprising culturing in the presence of a subject substance a test cell expressing a fusion protein comprising an APP modified protein according to Item 1 and a detectable secretory protein, and measuring detectable secretory proteins which were cleaved by β-secretase and secreted outside the cell.
Item 10. A method for examining the cytotoxicity of a subject substance according to any one of Items 7 to 9, the method comprising performing a cell viability test of the test cell cultured in the presence of the subject substance.
Item 11. An APP modified protein according to Item 1, wherein Swedish familial Alzheimer's disease mutations are introduced to the protein.
Item 12. A fusion protein comprising an APP modified protein according to Item 1 and a detectable secretory protein, wherein Swedish familial Alzheimer's disease mutations are introduced to the fusion protein.
Item 13. An APP modified protein according to Item 1, wherein the protein has an amino acid sequence of any one of SEQ ID Nos. 2 to 6.
Item 14. An APP modified protein according to Item 1, wherein the protein has an amino acid sequence of any one of SEQ ID Nos. 7 to 11.
Item 15. A fusion protein comprising an APP modified protein according to Item 1 and a detectable secretory protein, wherein the fusion protein has an amino acid sequence of any one of SEQ ID Nos. 12 to 16.
Item 16. A fusion protein comprising an APP modified protein according to Item 1 and a detectable secretory protein, wherein the fusion protein has an amino acid sequence of any one of SEQ ID Nos. 17 to 21.
Item 17. An APP modified protein according to Item 1, wherein the protein has the following amino acid sequence: 
Lys-Met-Asp-Ala-Glu-Phe-Arg-His-Asp-Ser-Gly-Tyr-Glu-Val-His-
His-Gln-X-X-Val-Phe-Phe-Ala-Glu-Asp-Val-Gly-Ser-Asn-Lys-Gly-
Ala-Ile-Ile-Gly-Leu-Met-Val-Gly-Gly-Val-Val,
 wherein X represents any amino acid residue.
Item 18. An APP modified protein according to Item 1, wherein the protein has the following amino acid sequence: 
Asn-Leu-Met-Asp-Ala-Glu-Phe-Arg-His-Asp-Ser-Gly-Tyr-Glu-Val-
His-His-Gln-X-X-Val-Phe-Phe-Ala-Glu-Asp-Val-Gly-Ser-Asn-Lys-
Gly-Ala-Ile-Ile-Gly-Leu-Met-Val-Gly-Gly-Val-Val,
 wherein X represents any amino acid residue.
Item 19. A fusion protein comprising an APP modified protein according to Item 17 or 18 and a detectable secretory protein. Item 20. A nucleic acid molecule which encodes a protein according to any one of Items 11 to 19.
Item 21. A test cell which expresses an APP modified protein according to any one of Items 11, 13, 14, 17, and 18.
Item 22. A test cell which expresses a fusion protein according to any one of Items 12, 15, 16, and 19.
Item 23. A method for examining whether or not a subject substance affects β-secretase activity, the method comprising culturing in the presence of the subject substance a test cell according to Item 22, and measuring detectable secretory proteins which were cleaved by β-secretase and secreted outside the cell.
Item 24. A method for examining whether or not a subject substance affects β-amyloid production, the method comprising culturing in the presence of the subject substance a test cell according to Item 22, and measuring detectable secretory proteins which were cleaved by β-secretase and secreted outside the cell.
Item 25. A method for screening a substance useful as an active ingredient for a therapeutic agent or prophylactic agent for Alzheimer's-type dementia, the screening method comprising culturing in the presence of a subject substance a test cell according to Item 22, and measuring detectable secretory proteins which were cleaved by β-secretase and secreted outside the cell. Item 26. A method for examining the cytotoxicity of a subject substance according to any one of Items 23 to 25, the method comprising performing a cell viability test of the test cell cultured in the presence of the subject substance.

Hereinafter, the present invention is described inmore detail.

### β-amyloid precursor protein (APP) modified protein

The β-amyloid precursor protein (APP) modified protein of the invention is a protein which is characterized by containing a β-secretase cleavage site in APP and which is modified by an amino acid mutation or mutations which do not affect β-secretase cleavage.

Here, using APP695 as an example, the β-secretase cleavage site is a site between a methionine residue at the 596 position and an aspartic acid residue at position 597 (see Fig. 1). Again, using APP695 as an example, the α-secretase cleavage site is a site between a lysine residue at position 612 and a leucine residue at 613 position.

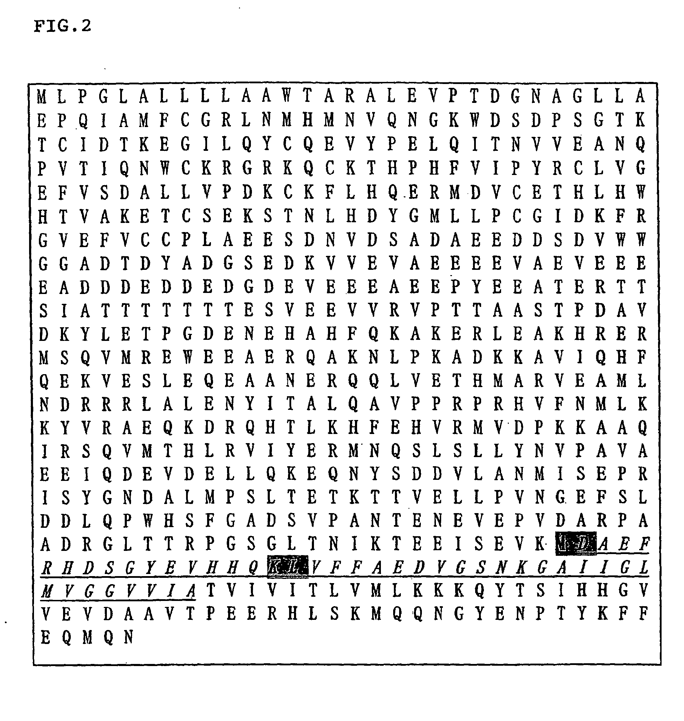

The amino acid sequence of APP695 is shown in SEQ ID No. 1 of Fig. 2 and a sequence list. The representation of amino acids, peptides, nucleotide sequences, nucleic acids and the like by abbreviations in this description is in conformity with the rules recommended by the IUPAC-IUB, *"Guidelines for Writing Descriptions Containing Nucleotide Sequences or Amino Acid Sequences"* (edited by the Japanese Patent Office), and the conventions relating to the use of codes or symbols in the art.

A number of molecular species of APP in addition to APP 695, such as APP751 and APP770, are known to exist. They are identical to each other in the α-secretase and β-secretase cleavage sites, and thus APP695 is used to describe the invention. The APP used for the APP modified protein of the invention, however, is not limited to APP695.

In the APP modified protein of the invention, there is no limitation to the length of the amino acid sequence at the N-terminus to the extent that a region required for exhibiting the above characteristics is contained and the β-secretase cleavage is not affected. All of the amino acid sequences at the C terminus are not necessarily required to the extent that the β-amyloid production caused by γ-secretase generated after the β-secretase region is cleaved is not affected.

Taking APP695 as an example, the region of positions 593 to 648 of the APP695 is required to demonstrate the above characteristics.

596 and 597 positions as a β-secretase recognition site need to be contained in the region. Since it has been reported that about 4 residues at the N terminus are necessary for the β-secretase to recognize APP695 as a substrate (Neuron, 14, 661-670 (1995)), the N terminus should start with at least position 593. 612 and 613 positions as the α-secretase site are also necessary. When the β-amyloid region (from 597 to 638) for confirming the production amount of the β-Amyloid is included, positions 593 to 638 are required. Since the APP is a type I membrane protein, positions 625 to 648 of a transmembrane region are required so that the APP may be normally fixed to a membrane and maybe undergo processing in the fixed state. This is why positions 593 to 648, which include these positions, are mentioned as an example.

Specific examples of proteins containing such a region include a protein having the C terminal region containing posit ions 581 to 695 of the amino acid sequence of APP695.

In the modified protein of the invention, an amino acid mutation or mutations which do not affect β-secretase cleavage and do not cause α-secretase cleavage is referred to as an amino acid mutation or mutations which do not cause α-secretase cleavage, and such an amino acid mutation or mutations do not eliminate nor reduce β-secretase cleavage activity.

Such an amino acid mutation or mutations are caused by deletion, substitution, addition, etc., of the amino acid at, for example, the α-secretase cleavage site.

There is no limitation to such an amino acid mutation or mutations to the extent that the mutation or mutations do not affect β-secretase cleavage nor cause α-secretase cleavage.

Particularly preferable examples of amino acid mutations include an amino acid mutation in which the leucine residue at position 17 counting from the amino acid at the N terminal β-amyloid is substituted by glutamic acid, proline, alanine, or lysine, or an amino acid mutation in which the lysine residue at position 16 is substituted by tryptophan.

Specific examples of proteins having the above amino acid mutation include an APP modified protein which has an amino acid sequence of any one of SEQ ID Nos. 2 to 6.

Further, in order to make an attack of β-secretase easier, the generally-known APP amino acid mutation or mutations in the hereditary Alzheimer's disease may be introduced into the modified protein of the invention. Specific examples of the APP amino acid mutation or mutations in the hereditary Alzheimer' s disease include a Swedish familial Alzheimer's disease APP mutations.

Specific examples of proteins into which such a mutation or mutations are introduced include modified proteins having an amino acid sequence of any one of SEQ ID Nos. 7 to 11.

The APP modified protein of the invention may be obtained by modifying a gene encoding an APP protein to a DNA sequence that encodes the amino acid sequence of the modified protein, introducing the mutated gene into a suitable expression vector, to be expressed in a host cell. Subcloning, etc. may be appropriately performed to acquire the target gene in each step. For example, an insertion of the target gene into a suitable cloning vector, and a screening of the target gene by a routine procedure may be conducted to increase the amount of the gene itself.

The selection of codons in the DNA molecule corresponding to the amino acid of an APP modified region is not limited, and any combination can be accepted to the extent that the DNA sequence encodes the amino acid sequence. The codons can be selected in a routine manner. For example, codons can be selected with reference to the codon usage frequencies in the host to be employed.

The target DNA molecular arrangement may be acquired by the DNA amplification method using PCR, for example. The primers used when PCR is employed can be suitably determined based on the sequence information of the target amino acid mutation or mutations, and can be synthesized according to a routine method.

The amplified DNA fragments can be isolated and purified by a routine method, such as gel electrophoresis, molecular sieving, etc.

The base sequence of the nucleic acid molecule of the invention can be determined according to the dideoxy method, the Maxam-Gilbert method, etc., or determined simply by using a commercially-available sequencing kit, etc.

The protein is produced based on the sequence information of the DNA molecule provided by the above DNA molecule according to a common gene recombination technique (see Sambrook, J., Frisch, E.F., and Maniatis, T., Molecular Cloning, A Laboratory Manual, 2nd Edition, Cold Spring Harbor Laboratory Press, 1989; Current Protocols in Molecular Biology, John Wiley Sons, Inc. 1998., etc.)

More specifically, the protein is produced by preparing a recombinant DNA (expression vector) in which a DNA molecule coding the desired protein can be expressed in a host cell, introducing the recombinant DNA into the host cell to be transformed, culturing the transformant, and subsequently collecting such proteins from the obtained culture.

Specific examples of host cells include cells derived from prokaryotes, cells derived from eukaryotes, etc.

Specific examples of those derived from prokaryotes include those derived from colibacillus, Bacillus subtilis, etc. Preferable examples of colibacillus are those belonging to the genus *Escherichia,* such as *Escherichia coli* K12 strain. Preferable examples of Bacillus subtilis are Bacillus strains, such as Bacillus subtilis Marburg 168.

When prokaryotic cells are used as a host cell, a vector which is replicable in the host cell is used, andapromoter, SD (Shine and Dalgarno) base sequence, and an initiation codon (e.g., ATG), which is necessary for initiation of the protein synthesis, are provided upstream of the gene in the vector, so that the DNA molecule may express.

Various known vectors can be used as the vector mentioned above, and plasmids derived from Escherichia coli, such as pBR322, pBR325, pUC12, pUC13, etc., are generally used. Examples of the vectors for use in expression systems using E. coli include pGEX-4T (Amersham Pharmacia Biotech), pMAL-C2. pMAL-P2 (New England Biolabs), pET21, pET21/lacq (Invitrogen) and pBAD/His (Invitrogen).

There is no limitation to usable promoters. Any promoter can be used. When a strain of the genus Escherichia is used as the host, tryptophan (trp) promoters, lpp promoters, lac promoters, recA promoters, PL/PR promoters, etc., are preferably used. When the host is a strain of the genus Bacillus , SP01 promoters, penP promoters, etc., are preferably used.

Usable cells derived from eukaryotes are cells derived from mammal, for example, and specific examples of such cells include the monkey cell line COS [Cell, 23: 175 (1981)], Chinese hamster ovary cells and a dihydrofolate reductase-defective cell line thereof [J.Exp.Med., 108: 945 (1958). Proc. Natl. Acad. Sci., 77:4216 (1980), NIH3T3 cells (J.Viol., 4:549 (1969)), Jurkat cells (J. Immunol., 133:123 (1984), etc. Cells of yeast belonging to the genus Saccharomyces, etc., are preferably used.

When cells derived from eukaryotes are used as the host cell, vectors having a promoter upstream of the DNA molecule to be expressed, an RNA splice site, a polyadenylation site and a transcription termination sequence are usually used as the expression vector. This vector may also have a replication origin as required. A specific example of the expression vector includes pSV2dhfr harboring an early promoter of SV40 [Mol. Cell. Biol., 1: 854 (1981)]. In addition to the above, various known commercially available vectors may be used. Examples of commercial vectors which are used in expression systems using animal cells include vectors for animal cells, such as pCI (Promega), pIND (Invitrogen), pcDNA3.1/His (Invitrogen), pEF1/V5-His (Invitrogen), etc., and vectors for insect cells, such as pFastBac HT (GibciBRL), pAcGHLT (PharMingen), pAcS/V5-His, pMT/V5-His and pMT/Bip/V5-his (all Invitrogen), etc.

Examples of preferable promoters for use when animal cells are used as the host cell include SV40-derived promoters, retrovirus promoters, metallothionein promoters, heat shock promoters, cytomegalovirus promoters, and SRα promoters, adenovirus promoters, the elongation factor promoters, etc. When a yeast is used as the host, pH05 promoters, PGK promoters, GAP promoters, ADH promoters, etc., are preferably used.

Introduction of expression vectors into the host cell and transformation caused by the introduction can be carried out without limitation in accordance with various general methods, such as an electroporation, liposome method, calcium phosphate method, DEAE dextran method, etc.

The protein of the invention can be obtained by culturing the host cells, and appropriately isolating and purifying the produced proteins.

The protein thus obtained can be used per se for screening a compound affecting β-secretase.

When the protein is produced using cells derived from eukaryotes, isolated proteins or the whole cell may be used.

In the coexistence of the test substance and the protein (or cells expressing the protein), β-secretase is acted and its influence is measured, thereby screening a substance which affects β-secretase or the production of β-amyloid.

Proteins can be detected by Western blotting, ELISA method, or the like, using an antibody which recognizes the amino acid sequence of APP, for example.

### Fusion protein

The fusion protein of the invention denotes a protein obtained by fusing proteins other than the APP modified protein, e.g., a detectable secretory protein, into the N-terminal protein of the above-described APP modified protein of the invention.

Here, the detectable secretory protein denotes a protein or a peptide which does not contain the localization sequence for each intracellular tissue in the amino acid sequence, and which is released or emitted outside the cell to demonstrate this function. More specifically, this refers to a protein which is cleaved at the C terminal region of the protein by β-secretase and secreted outside the cell.

Preferable examples of detectable secretory proteins include a protein which can be easily detected using a substrate like an enzyme; a protein which has physical properties and thus the amount secreted outside the cell or into the culture supernatant can be quantatively measured using an instrument, a reagent, etc.: or a protein which demonstrates a bioactivity outside the cell. In addition to the above, usable proteins include those in which the bioactivity resulting from the physiologically active protein in the culture supernatant can be determined, or in which the amount of the secretory protein in the culture supernatant can be generally determined using the antibody against the secretory protein with ELISA or Western blotting, for example.

More specifically, secretory alkaline phosphatase, β-galactosidase, peroxidase, luciferase, etc., can be mentioned. These can be quantified as the amount of enzyme activity by, for example, adding an enzyme substrate in the culture supernatant.

Other examples include a green fluorescence protein (GFP), aequorin, immunoglobulin, growth hormone, histidine tag, c-myc tag, Flag tag, hemagglutinin (HA) tag, glutathione S transferase (GSP) tag, T7 tag, V5 epitope tag, X-press tag, BE11 tag, etc.

Furthermore, a physiologically activating protein group belonging to cytokine, for example, interleukin 2, can be mentioned. The protein released outside the cell can be detected by measuring the bioactivity in a culture medium or by measuring with a commercially-available ELISA kit.

Although these secretory proteins are desirably produced and secreted as a secretory-type protein having an original signal peptide, they can be secreted using a signal peptide of the secretory protein of APP and other secretory proteins.

These secretory proteins can be partially modified, deleted, and inserted to the extent that they do not significantly affect the detected enzyme activities, immunoreactivity, bioactivity, etc.

The APP modified protein encompassed in the fusion protein of the invention is a protein subjected to the above-described amino acid mutation or mutations such that the β-secretase cleavage site is contained, the β-secretase cleavage is not affected, and the α-secretase cleavage does not arise.

There is no limitation to the amino acid mutation or mutations to the extent that the amino acid mutation or mutations do not affect the β-secretase cleavage nor cause the α-secretase cleavage. Particularly preferable is an amino acid mutation in which the leucine residue at position 17 counting from the N terminal amino acid of β-amyloid is substituted by glutamic acid, proline, alanine, or lysine, or the lysine residue at position 16 is substituted by tryptophan.

Specific examples of fusion proteins of the invention include those having an amino acid sequence of any one of SEQ ID Nos. 12 to 16.

Further, in order to make the β-secretase attack easier, a generally-known APP amino acid mutation or mutations in hereditary Alzheimer's disease, such as a Swedish familiar Alzheimer's disease APP mutations, may be introduced into the modified protein of the invention.

Specific examples of fusion proteins include those having an amino acid sequence of any one of SEQ ID Nos. 17 to 21.

In the fusion protein, the N terminal region of the APP modified protein may be extended, or a spacer may be suitably inserted between a secretory protein and an APP modified protein, as required, so that the β-secretase cleavage site in the APP does not have a steric hindrance that affects the attack of the β-secretase.

The fusion protein can be produced from a gene which encodes the above-described APP modified protein of the invention and a gene which encodes secretable protein according to the genetic recombination procedure in the same manner as in the above-described production process for the APP modified protein of the invention.

### Test Cell

Hereinafter, the test cell of the invention which stably expresses the above described APP modified protein or fusion protein of the invention is described.

The test cell used in the invention denotes a cell used for a test to measure the effect, toxicity, etc., of a compound and to evaluate the measurement results.

The test cell can be produced using the same genetic recombination procedure and materials (expression vector) as previously described.

In the cell used as the test cell, it is preferable that the gene expression mechanism, protein expression mechanism, and secretion mechanism are similar to human components or human mechanisms. As the cell expressing a fusion protein, it is preferable to use, for example, a cell with a mechanism such that secretory proteins are released outside the cell after cleavage at the APP site. The cell which has a rapid growth rate and maintains the stability even after subculturing is preferable. These include, for example, human-derived cells or mammalian-derived cells such as CHO-K1 cell (J. Exp.Med., 108: 945 (1958), a Hela cell (Cancer Res., 12: 264 (1952)), a NIH3T3 cell (J. Viol. , 108: (1958)), a Jurkat cell (J. Immunol., 133: 123 (1984)), a 293 cell (J. Gen.Viol., 36: 59 (1977)), etc.

The following kind of procedure may be applied to obtain a cell strain with stable expression as a test cell. For example, usable is a method comprising integrating a drug resistance gene, such as aminoglycoside phosphotransferase (Th5neo), hygromycin B phosphotransferase (hygr), xanthin guanine phosphoribosyl transferase (Eco-gpt), dihydrofolate reductase (dhfr), etc., into an expression vector, introducing the target gene-containing expression vector into a cell, and cultivating it in a medium containing the selective drug, such as G418, hygromycin, a HAT, and a methotrexate. For example, pSV2neo, pRSVneo, pHyg, pSV2gpt, or pSV2dhfr is used as an expression vector.

In order to screen a highly expressive cell strain, secreted proteins in the culture supernatant or fusion proteins may be measured. For example, the enzyme activity in the culture supernatant may be measured. Detection using the antibody recognizing the C terminal region of APP is performed according to Western blotting or ELISA. Measurement by the Northern blotting method or RT-PCR method is performed at the gene expression level.

### Measurement method

The measurement method of the invention comprises processing the above-described test cell with a subject substance, and measuring detectable secretory proteins which are cleaved at the β-secretase cleavage site and are secreted outside the cell.

The following processes can be adopted. The test cells are initially suspended in a culture medium, and are then seeded on a suitable culture plate. The seeded cells are incubated, as required, for a given period of time until they are stabilized. Any plate can be used by considering the number of seeded cells and the measurement sensitivity of the secretory proteins. For example, a 96-well plate is suitable.

The subject substance is then added to an appropriate solvent and dissolved, and the solution is added to a plate as a fixed concentration. In this case, amphiphilic solvents, such as DMSO, may be used when the subject substance is a fat-soluble compound or an insoluble compound. Water-soluble solvents, such as physiological saline, can be used when the subject substance is a hydrophilic compound. The concentration of the solvent used is preferably adjusted to a degree such that the cells are not adversely affected. After the subject substance is added, incubation is conducted for a given-period-of-time. The incubation period can be determined without limitation according to a test system to the extent that the subject substance is incorporated into a cell and sufficiently reacted with a target molecule.

After the incubation is complete, a given quantity of the culture supernatant is taken, and the quantity of the secretory proteins in the supernatant is measured according to the properties. When the secretory protein is, for example, an enzyme, a substrate is added to measure the enzyme activity. When the secretory proteins have characteristic physical properties, such as a specific absorption spectrum, an absorbance meter is used for measurement. The target proteins may be quantified by monitoring them through a separation operation using HPLC, etc., as required. When a secretory protein is a physiologically active protein, such as cytokine, the bioactivity resulting from the proteins in the culture supernatant may be measured. The measurement and quantification may be made using the commercially-available ELISA kit, etc.

The existence form of a fusion protein expressing in a cell may be monitored. More specifically, it can be directly confirmed whether or not a fusion protein is cleaved at the β-secretase cleavage site by measuring the amount of 99-amino acid C-terminal fragment (C99) which is produced by the β-secretase cleavage. For example, after lysis of cell, C99 can be detected and quantified using an antibody which recognizes C99 according to Western blotting or ELISA. The above-described measurement method of C99 is applicable not only to the expression cell of a fusion protein but to the expression cell of an APP modified protein. Therefore, the cell can be used for screening a compound affecting β-secretase activity, a compound affecting β-amyloid production, or a substance usable as the active ingredient for a therapeutic or prophylactic agent of Alzheimer's-type dementia.

The effect of the subject substance on the β-secretase activity or the β-amyloid production can be determined based on a comparison between the measurement value obtained when the subject substance is added according to the above-described measurement method using the modified protein of the invention and the base value obtained when the subject substance is not added (solvent only). More specifically, when the measurement value is lower than the control value, the β-amyloid production is judged to be suppressed. In contrast thereto, when the measurement value is higher than the control value, the β-amyloid production is judged to be promoted.

Whether or not the subject substance affects the β-amyloid production is judged by performing statistical analysis, i.e., a significant difference test.

Whether or not the subject substance affects the β-secretase activity can be examined by the above-described procedure. Further, it is possible to screen the substance as a candidate for the active ingredient for a therapeutic or prophylactic agent of Alzheimer's-type dementia.

The measurement method of the invention may further comprise a step of examining whether or not the subject substance shows toxicity to a cell.

Whether or not the subject substance has toxicity to a cell can be judged by culturing the test cell with the subject substance, measuring the detectable secretory protein, and then measuring the survival degree of the cultured test cells. The number of survival cells can be determined by measuring the number of cells dyed by, for example, calcein AM. The number of dead cells can be determined by measuring the number of cells dyed by, for example, propidium iodide. Alternatively, the number of dead cells can be measured by counting the number of cells dyed by trypan blue. Alternatively, the number of survival cells may be determined by colorimetry measuring the formazan dye of the tetrazolium salt (MTT, MTS, WST-1, WST-8, etc.) produced by the survival cells. The number of dead cells may be determined by measuring the lactate dehydrogenase (LDH) which has flowed into the culture supernatant due to cell death, such as apotosis. Of course, these determination methods are not limited to the above.

### BRIEF DESCRIPTION OF DRAWINGS

Fig. 1 schematically illustrates an APP structure and APP processing.
Fig. 2 shows an amino acid sequence of APP 695. The underlined portion shows the amino acid sequence of β-Amyloid (1-42). The shaded portions, MD and KL, denote the sites to be cleaved by β-secretase and α-secretase, respectively.
Fig. 3A shows the result of Western blotting. This shows that the amino acid substitution at the α-secretase cleavage site (position 17) in APP affects the β-secretase cleavage.

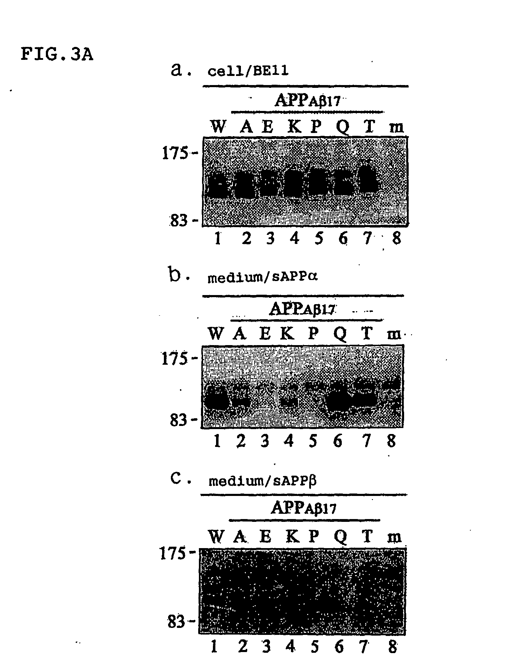

Fig. 3A(A) shows the result of a process comprising carrying out electrophoresis of 8% SDS-PAGE after cytolysis, and conducting Western blotting. A full-length APP was detected by a monoclonal antibody against a BE11 epitope tag.
Fig. 3A(B) shows the result of a process comprising electrophoresing a culture supernatant, and conducting Western blotting in the same manner as in the above A. A monoclonal antibody (anti-sAPPα antibody) which recognizes the amino acids of the region between the α-secretase and β-secretase cleavage sites was used for detection.
Fig. 3A(C) shows the result of a process comprising electrophoresing a culture supernatant, and conducting Western blotting in the same manner as in the above B. An antibody (anti-sAPPβ antibody) which recognizes the APP N terminal region from N terminus to position 596 was used for detection.

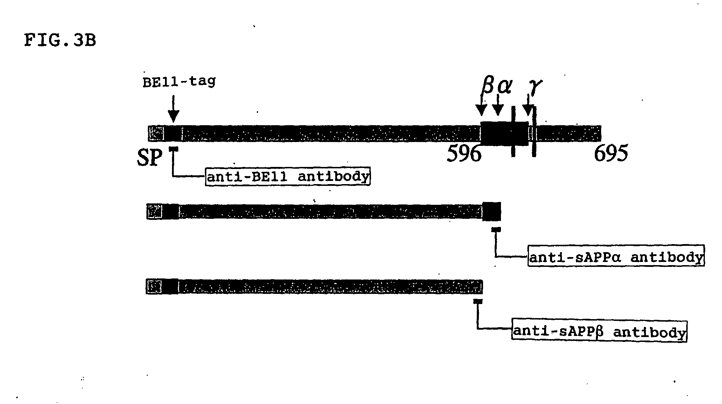

Fig. 3B schematically illustrates an antibody recognition site.

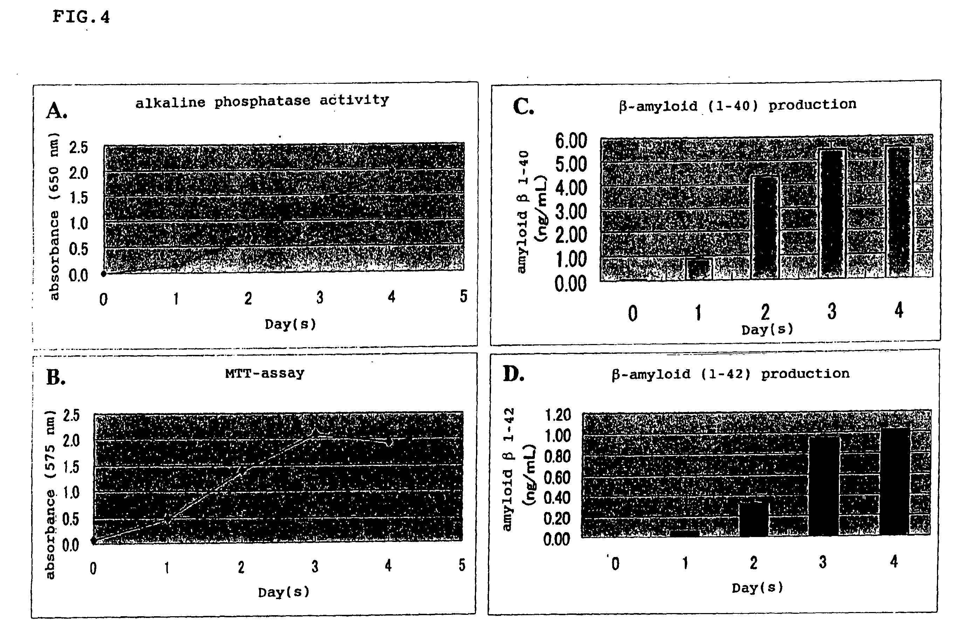

Fig. 4A shows the alkaline phosphatase activity of the culture supernatant of a CHO K1 cell line stably expressing SEAP-APPsw(CHO/SW).
Fig. 4B shows cell proliferation of a CHO K1 cell line stably expressing SEAP-APPsw (CHO/SW) by MTT assay.
Figs. 4C and 4D show the production amount of β-amyloid in the culture supernatant.

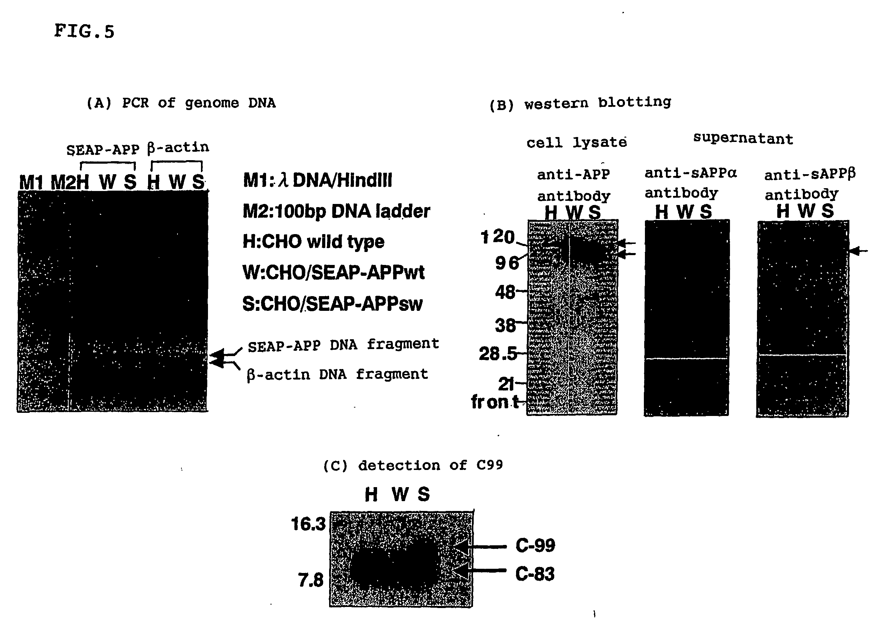

Fig. 5 (A) shows confirmation by PCR of whether or not the gene which encodes the fusion protein of the invention is introduced into the cell in the selected clone.
Fig. 5(B) shows confirmation by Western blotting of whether or not fusion proteins are actually synthesized, and are then cleaved only at the target site to be secreted in the culture supernatant in the selected clone.
Fig. 5(C) is a view showing the result of confirming an intracellular C99 by Western blotting. The obtained result shows whether or not fusion proteins are actually synthesized, and are then cleaved at the target site in the selected clone.

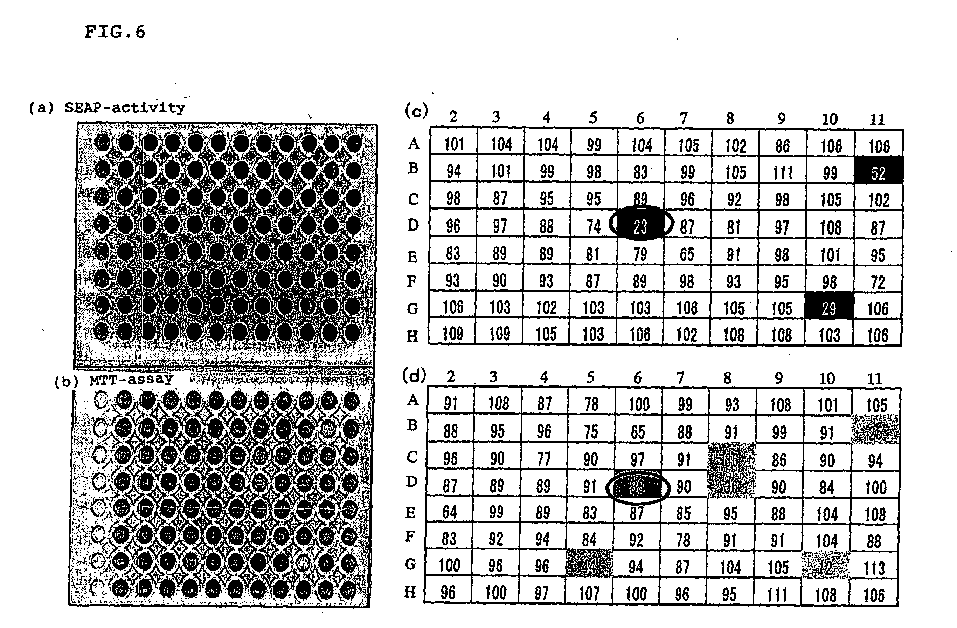

Fig. 6 shows examples the result of the screening in accordance with the invention.
Fig. 6 (a) shows the result of determining the alkaline phosphatase activity in the culture supernatant on 650 nm by colorimetry.
Fig. 6 (b) shows the measurement result of the number of cells by MTT assay.
Fig. 6 (c) shows the evaluated alkaline phosphatase activity in numerical values for the case in which the subject substance-free condition is set to 100.
Fig. 6 (d) shows the evaluated number of cells by MTT assay in numerical values for the case in which the subject substance-free condition is set to 100.

### BEST MODE FOR CARRYING OUT THE INVENTION

Hereinafter, the present invention is described with reference to Examples, however it is not limited thereto.

### Example 1: Construction and expression of APP modified protein gene

BE11-hAPP695 was used as a starting material which was produced by adding a BE11 epitope tag immediately after a signal peptide of the N-terminus of a human-type APP gene (Fig. 2) and incorporating it into a vector. With respect to the amino acid at the N-treminus of the α-secretase cleavage site of APP695 (lysine at position 16 of β-amyloid) or the amino acid at the C-terminus (leucine at position 17 of β-amyloid), tryptophan was introduced into position 16 and alanine, glutamic acid, lysine, proline, glutamine, and threonine were introduced into position 17 by the site-directed mutagenesis (QuickChange, Stratagene). A construct was produced in which these mutated APPs and wild-type APPs are respectively incorporated into a pCIneo mammalian expression vector (Promega), and the construct was then transfected to a COS7 cell by a liposome method (DOTAP, Boeringer Manheim), to achieve transient expression. Forty-eight hours after transfection, the cell and culture supernatant were collected, and the expressed proteins and the processing were analyzed by 8% SDS-PAGE/Western blotting. The expression of a cell membrane-associated full-length APP confirmed using the monoclonal antibody (Mab BE11) against BE11. The expression level was standardized, and then α-secretase cleavage products (sAPPα) and β-secretase cleavage products (sAPPβ) which were secreted in the culture supernatant (medium) were detected and quantified using a specific antibody against the respective products. The results are shown in Fig. 3 and Table 1.

**Table 1**

| | α-cleavage | β-cleavage |
|---|---|---|
| Trp (position 16) | ↓↓ | → |
| Ala (position 17) | ↓ | → |
| Glu (position 17) | 0 | → |
| Pro (position 17) | 0 | → |
| Lys (position 17) | ↓↓ | ↑ |

In Table 1, " ↓ " indicates a reduced amount of secretase cleavage product, "→" indicates an unchanged amount of each secretase cleavage product, " ↑ " indicates an increased amount of secretase cleavage product, and "0" indicates no detection of each secretase cleavage product.

In Fig. 3, the reference characters, A, E, K, P, Q, and T denote amino acid mutated to alanine, glutamicacid, lysine, proline, glutamine, and threonine, respectively. The left end "W" denotes a wild type and the right end "m" means "mock", i.e., a vector only.

As can be seen from the result shown in Fig. 3 or Table 1, the amount of α-secretase cleavage product decreased when position 17 was replaced with alanine. The amount of α-secretase cleavage product sharply decreased when position 16 was replaced with tryptophan. No α-secretase cleavage product was detected when position 17 was replaced with glutamic acid or proline.

The β-secretase cleavage was not affected by these mutations.

When position 17 was replaced with lysine, the amount of α-secretase cleavage product decreased and the amount of β-secretase cleavage product slightly increased.

It was confirmed based on the above results that the protein introducing these mutations of the invention was effectively used for efficiently screening substances which affect β-Amyloid production.

### Example 2: Construction of a fusion protein gene and formation of a test cell

### (1) Construction of a fusion protein gene

A fusion protein was constructed using an APP modified protein which was mutated by replacing position 17 of β-amyloid with glutamic acid.

cDNA (pSEAP2-Basic (Clontech, Cat.No.6049-1)), which encodes secretory alkaline phosphatase (SEAP), was treated with EcoRI and XbaI to produce an EcoRI-XbaI fragment. However, the region corresponding to the last ten amino acids of the SEAP coding region was excluded.

Subsequently, EcoRI-XbaI fragment was isolated by PCR using a human APP modified protein cDNA fragment, i.e., a C terminal region (581 to 695 amino acids from the N-terminus of APP). The following primers were used:

Sense: TCTAGACCAGGTTCTGGGTTGACAAATATC (including XbaI)

Antisense: CCGGATCCCTAGTTCTGCATCTGCTCAAAGAACTTGTA
(including NotI).

A gene encoding the APP modified protein of the invention which was mutated by replacing position 17 of β-amyloid with glutamic acid was used as a template DNA. As a reaction solution, 25 µl of a solution of the following composition was used (20 mM Tris-HCl with pH 8.0 containing 10 mM KCl, 10 mM(NH₄)₂SO₄, 2 mM MgSO₄, 0.1% Triton X-100, 400 µM NTP, 0.5 µM Primer (each sense and antisense), 10 ng template DNA, 0.01% BSA, and 0.25 unit Ventr'q DNA polymerase (NEB, Cat.No.254L)). Amplification was conducted under the reaction condition at 94°C for 30 seconds, 55°C for 1 minute, and 72°C for 2 minutes, and 25 cycles were repeated. The amplified PCR fragments were cloned using pT7 blue-3 (Novagen Inc., Cat. No. 70029-3). E.coli DH5a (Toyobo, Cat. No. DNA-901) was used for the transformation. The above-described process produces pT7 blue-3/cAPPE.

Subsequently, the following processes were conducted to insert Swedish type mutations. More specifically, a DNA adapter containing BglII and an EcoRI site was initially produced. The following sequences were used as oligonucleotide sequences:

Sense: GATCTCTGAAGTGAACCTGGATGCAG

Antisense: AATTCTGCATCCAGGTTCACTTCAGA.

The DNA adapter was inserted into pT7 blue-3/cAPPE, which was previously digested by BgIII and EcoRI, and subcloning was performed according to the routine procedure, producing pT7 blue-3/APPE.

Subsequently, pT7 blue-3/APPE was processed by XbaI and NotI according to the routine procedure, and the XbaI-NotI fragment obtained was used to produce a fusion protein.

With respect to SEAP, pSEAP2-Basic was processed by EcoRI and XbaI, producing an SEAP EcoRI-XbaI DNA fragment.

The two types of DNA fragments, i.e., the pT7 blue-3/APPE XbaI-NotI DNA fragment and the SEAP EcoRI-XbaI DNA fragment were ligated into expression vector pEF1/V5-His which was previously processed by EcoRI and NotI, and the construction of a fusion protein expression gene was completed.

### (2) Formation of a test cell line for a fusion protein stable expression

The expression vector was transfected using a transfection reagent of Polyfect™ (Qiagen, Cat. No. 301105) to Chinese hamster ovary cells (CHO-K1, ATCC CCL61), which was cultivated on a 60 mm-culture dish. The culture was carried out using a culture medium which was obtained by adding cow fetus blood serum (FBS) (JRH Bioscience, Cat. No. 12103-789, Lot. No. 9K2087) to a DMEM/F12 culture medium (Sigma Chemical Co., Cat. No. D8062) to bring the final concentration to 10%. Forty-eight hours after culture initiation, the processed cells were suspended in the above-described culture medium containing Geneticin (500 µg/ml), and thereafter the resultant cells were seeded to a 96-well microplate at ratios of 1, 10, and 100 cells/well. Two weeks later, the wells containing a positive clone were screened. The alkaline phosphatase activity of the culture supernatant was determined using the alkaline phosphatase substrate (Blue Phos™, KPL, Cat. No. 50-88-00). Clones showing stable expression were screened based on the determined alkaline phosphatase activity.

It was confirmed in the obtained clone (CHO/sw) that cells proliferated with time (Fig. 4B), and the alkaline phosphatase activity in the culture supernatant increased with time (Fig. 4A).

The fusion protein expression cell (CHO/sw) established thus secreted alkaline phosphatase as a β-secretase cleavage product in the culture supernatant as the cell proliferation and culture period proceeded. It was clarified that the activity of the secreted alkaline phosphatase correlated with the production amount of β-amyloid secreted. It was confirmed based on the above result that a target cell line which allows detection of the β-amyloid production amount by determining the activity of secreted alkaline phosphatase was successfully produced.

Further, it was confirmed that the production amount of β-amyloid correlated with the alkaline phosphatase activity by measuring the β-amyloid in the culture supernatant by ELISA (Fig. 4 C and D). The alkaline phosphatase activity and cell proliferation by MTT were determined by a procedure that is described later. β-amyloids (1-40) and (1-42) were determined using a commercially available kit (Immuno-Biological Laboratories Co., Ltd., Code Nos. 17714 and 17712).

### (3) Confirmation of a gene introduced into a fusion protein

Whether or not a gene was actually transferred was further confirmed. The gene-introduced CHO-K1 cell, which was cultured to be confluent state on a 100-mm dish was suspended in 500 µl of a digestion buffer (10 mM Tris-HCl with pH8.0 containing 100 mM NaCl, 25 mM EDTA, 0.5% SDS, 0.1 mg/ml protein-kinase K (proteinase K)) and was incubated at 50°C overnight. The cell lysate was then extracted with phenol chloroform (1:1), isopropanol was added in equal proportions to an aqueous layer, and DNA was collected by centrifugation at 1,700 × g for 2 minutes. Subsequently, DNA was suspended in 0.5 mg/ml of TE buffer solution (Tris-HCl with pH8.0 containing 1 mM EDTA), and was completely dissolved at 60°C. PCR was conducted using the prepared DNA (2.5 µg) as a template using Takara Ex Taq (Takara, Cat. No. RR001A) and 0.5 µM of each primer described later. The PCR was conducted at 94°C for 30 seconds, 60°C for 1 minute, and 72°C for 2 minutes, and 30 cycles were repeated. Primers for the fusion protein are as follows:

Sense: TCTAGACCAGGTTCTGGGTTGACAAATATC

Antisense: CCGGATCCCTAGTTCTGCATCTGCTCAAAGAACTTGTA.

Primers for β-Actin are as follows:

Sense: TTTGAGACCTTCAACACCCC

Antisense: AGGAAAGGAAGGCTGGAAGA.

The β-Actin gene was used as a control gene.

The results are shown in Fig. 5(A). In Fig. 5(A), the reference character "S" denotes a cell line with a fusion protein (SEAP-APPsw) gene to which Swedish mutations were added, "W" denotes a cell line with a fusion protein (SEAP-APPwt) gene to which no Swedish mutations were added, and "H" denotes a non-transgenic CHO cell line.

As can be seen from the result shown in Fig. 5(A), it was confirmed that the fusion protein gene (SEAP-APP) was introduced in the fusion protein expression cell line (S and W in the drawings).

Next, it was confirmed by Western blotting whether or not the obtained clone expressed the target fusion protein.

A CHO-K1 cell cultured to be a confluent state on a 100-mm dish was suspended in 1 ml of a cytolysis buffer (50 mM Tris-HCl with pH8.0 containing 150 mM NaCl, 0.1% SDS, 1% NP-40, 0.5% Chaps, 10 mg/ml aprotinin, 10 mg/ml leupeptine), and the suspension was intermittently vortexed for 30 minutes on ice. Cytolysis was centrifuged at 4°C for 10 minutes at 14,000 rpm, and the supernatant was used as a sample for Western blotting. Subsequently, a cell extract and 16 µl of a culture supernatant were mixed into a sample preparation buffer (0.624 MTris-HCl with pH6.8 containing 10mMEDTA, 10%SDS, 25% glycerol, 0.1% BPB, and 1% 2-mercaptoethanol), and the mixture was electrophoresed with 13% polyacrylamide gel electrophoresis (10×10cm slab gel). After the electrophoresis, the protein was transferred to a nitrocellulose membrane (Hybond™-ECL, Amersham Pharmacia Biotech, Cat. No. RPN3032D) using a semidry blotter (Trans-Blot SD, Bio Rad, Cat. No. 170-3940). Subsequently, the nitrocellulose membrane was blocked at room temperature for 1 hour in a Blotto-B buffer (PBS containing 1% BSA, 1% skim milk, and 0.05% tween-20), and was treated with the APP recognition antibody solution (at room temperature for 2 hours). The nitrocellulose membrane was sufficiently washed with a washing solution (PBS containing 0.05% Tween20), and treated with the secondary antibody (Peroxidase-conjugated anti-mouse IgG (Santa Cruz Biotechnology Inc. Cat. No. SC-2030) and Peroxidase-conjugated anti-rabbit IgG (Santa Cruz Biotechnology Inc. Cat. No. SC-2005)) at room temperature for 1 hour. A chemiluminescence detection kit (Super Signal'^{q}, Pierce Co., Cat. No. 34080) was used for the detection.

The anti-human APP antibody (C terminal portion recognition antibody, Sigma Chemical Co., Cat. No. A-8717), the anti-sAPPα antibody (Otsuka Pharmaceutical Co., Ltd.), and the anti-sAPPβ antibody (Immuno-Biological laboratories Co., Ltd.) were used as primary antibodies.

It was confirmed by the result of Western blotting for cell lysate (Fig. 5(B), left photograph) that APP was expressed at the desired position as indicated by the arrows. The upper band shows endogenous APP and the lower band shows a fusion protein. It was confirmed that a fusion protein was expressed in the fusion protein expression cell line with Swedish mutations (S) and in the fusion protein transgenic cell line with no Swedish mutations (W).

The result of Western blotting for the culture supernatant shows that no α-cleavage product was detected in any culture supernatant (Fig. 5(B), middle photograph).

In contrast, a β-secretase cleavage product was detected only in the fusion protein expression cell line (S) (Fig. 5(B), right photograph).

It was confirmed by the above result that the fusion protein expression cell line expressed the desired fusion protein at a protein level, and the fusion protein was subjected to preferential β-cleavage to be secreted outside the cell, as intended.

The C terminal region produced by the cleavage of APP was also confirmed. The Western-blotting sample (supernatant of a cell lysate) prepared as described above was concentrated using acetone. Specifically, to 167 µl of the Western-blotting sample was added cold acetone in the amount of 8 times the sample, the mixture was stirred, allowed to stand overnight at -20°C, centrifuged at 4°C at 14,000 rpm for 10 minutes, and an anti-human APP C terminus antibody (Sigma Chemical Co., Cat. N.A-8717) was detected as a primary antibody.

The fusion protein expression cell line (S) was subjected to preferential β-cleavage, and it was observed, as shown in Fig. 5 (C), the β-cleavage product, i.e., the C terminus 99 residue fragment (C99), increased in the cell. Since the α-cleavage product, C83, which was simultaneously observed, was similarly observed in the host cell, it was presumed to be a molecule produced by the processing of an endogenous APP.

As described above, it was confirmed that the test cell of the invention contained a β-secretase cleavage site; expressed a fusion protein containing a β-amyloid precursor protein (APP) modified protein which was subjected to an amino acid mutation without affecting the β-secretase cleavage and without causing α-secretase cleavage at the α-secretase cleavage site; and was subjected to the target processing (β-secretase cleavage) to secrete a detectable secretory protein. Further, it was clarified that the APP modified protein into which Swedish type mutations were introduced exhibited a high substrate specificity of β-secretase, and produced 5 to 10 times as much β-amyloid.

### Example 3: Screening of a compound using a test cell

### (1) Determination of effects on β-amyloid production

The cell obtained in Example 2, i.e., a stable cell line with a gene of fusion protein (SEAP-APPsw), was seeded to a 96-well microplate at 5,000 cells/well, a subject substance previously dissolved in DMSO was added thereto so that the final concentration became 5 µl/ml, and incubated under 5% CO₂ overnight (18 to 24 hours). Thereafter, 50 µl of the culture supernatant was transferred to a microplate for measuring the alkaline phosphatase activity, 50 µl of an alkaline phosphatase substrate (Blue Phos™, KPL, Cat. No. 50-88-00) was added and reacted for 1 hour, a reaction stop solution (Blue Phos™, KPL, Cat. No. 50-89-01) 100 µl was added, and 650-nm absorption was measured using a microplate reader (Labsystems Multiskan'q Multisoft) (Figs. 6a and 6c).

A subject substance which shows a significant difference in absorption between when it was added and when it was not added was regarded as a candidate substance for affecting β-amyloid production.

### (2) Determination of toxicity of a subject substance to cells

50 µl (1 mg/ml culture medium) of an MTT solution was added to each well of the microplate onto which the above-described test cells were seeded, and the reaction was conducted in a 5% CO₂ incubator for 4 hours. After the reaction, the supernatant was discarded, 100 µl of DMSO was added to each well and stirred, and 575-nm absorption was measured using a microplate reader (Labsystems Multiskan'q Multisoft) (Figs. 6b and 6d). A subject substance which showed absorption approximately equivalent to absorption when it was not added was judged to have no cytotoxicity.

The measurement results of Example 3 are shown in Fig.6. Figs. 6a and 6b are photographs of the actual microplates that were used. Fig. 6a shows the result of measuring alkaline phosphatase activity, and Fig. 6b shows the result of cytotoxicity by MTT assay. The first column of the plate shows a blank which does not contain any cells. The 12^{th} column shows a control solution which contains a cell but does not contain any subject substance. The 2^{nd} to 11^{th} columns show the cases where the subject substance was added.

The values in Figs. 6C and 6d show percentages of the absorbance obtained relative to the absorbance of the control. The value in each cell corresponds to that of the 2nd to 11th columns in the left-side photograph.

Based on the results, the decrease in alkaline phosphatase activity was observed in 6D, 10G, and 11B. Cytotoxicity was observed in 5G, 8C, 8D, 10G, and 11B. Considering these results overall, the 6D compound was screened as a compound which affects β-amyloid production, and has little cytotoxicity.

### INDUSTRIAL APPLICABILITY

The present invention provides a substance which can efficiently monitor β-amyloid production. Specifically, the present invention provides a β-amyloid precursor protein (APP) modified protein, a fusion protein containing the modified protein, a test cell expressing those proteins, etc. The use of such material allows the efficient screening of substances affecting β-amyloid production. Substances obtained by the screening serve as active ingredients for therapeutic or prophylactic agents of Alzheimer's disease.

As described above, the present invention particularly provides an excellent means for use in techniques for the prevention or treatment of Alzheimer's disease.

## Claims

1. A modified protein of β-amyloid precursor protein (APP), wherein (i) a β-secretase cleavage site is contained and (ii) one or more amino acids are mutated so that the β-secretase cleavage is not affected and α-secretase cleavage does not occur.

2. A nucleic acid molecule which encodes an APP modified protein according to Claim 1.

3. A test cell which expresses an APP modified protein according to Claim 1.

4. A fusion protein, comprising an APP modified protein according to Claim 1 and a detectable secretory protein.

5. A nucleic acid molecule encoding a fusion protein, comprising an APP modified protein according to Claim 1 and a detectable secretory protein.

6. A test cell expressing a fusion protein comprising an APP modified protein according to Claim 1 and a detectable secretory protein.

7. A method for examining whether or not a subj ect substance affects β-secretase activity, the method comprising culturing in the presence of the subject substance a test cell expressing a fusion protein comprising an APP modified protein according to Claim 1 and a detectable secretory protein, and measuring detectable secretory proteins which were cleaved by β-secretase and secreted outside the cell.

8. A method for examining whether or not a subject substance affects β-amyloid production, the method comprising culturing in the presence of the subject substance a test cell expressing a fusion protein comprising an APP modified protein according to Claim 1 and a detectable secretory protein, and measuring detectable secretory proteins which were cleaved by β-secretase and secreted outside the cell.

9. A method for screening a substance useful as an active ingredient for a therapeutic agent or prophylactic agent for Alzheimer's-type dementia, the screening method comprising culturing in the presence of a subject substance a test cell expressing a fusion protein comprising an APP modified protein according to Claim 1 and a detectable secretory protein, and measuring detectable secretory proteins which were cleaved by β-secretase and secreted outside the cell.

10. A method for examining the cytotoxicity of a subject substance according to any one of Claims 7 to 9, the method further comprising performing a cell viability test of the test cell cultured in the presence of the subject substance.

11. An APP modified protein according to Claim 1, wherein Swedish familial Alzheimer's disease APP mutations are introduced to the protein.

12. A fusion protein comprising an APP modified protein according to Claim 1 and a detectable secretory protein, wherein Swedish familial Alzheimer's disease APP mutations are introduced to the fusion protein.

13. An APP modified protein according to Claim 1, wherein the protein has an amino acid sequence of any one of SEQ ID Nos. 2 to 6.

14. An APP modified protein according to Claim 1, wherein the protein has an amino acid sequence of any one of SEQ ID Nos. 7 to 11.

15. A fusion protein comprising an APP modified protein according to Claim 1 and a detectable secretory protein, wherein the fusion protein has an amino acid sequence of any one of SEQ ID Nos. 12 to 16.

16. A fusion protein comprising an APP modified protein according to Claim 1 and a detectable secretory protein, wherein the fusion protein has an amino acid sequence of any one of SEQ ID Nos. 17 to 21.

17. An APP modified protein according to Claim 1, wherein the protein has the following amino acid sequence: 
Lys-Met-Asp-Ala-Glu-Phe-Arg-His-Asp-Ser-Gly-Tyr-Glu-Val-His-
His-Gln-X-X-Val-Phe-Phe-Ala-Glu-Asp-Val-Gly-Ser-Asn-Lys-Gly-
Ala-Ile-Ile-Gly-Leu-Met-Val-Gly-Gly-Val-Val,
 wherein X represents any amino acid residue.

18. An APP modified protein according to Claim 1, wherein the protein has the following amino acid sequence: 
Asn-Leu-Met-Asp-Ala-Glu-Phe-Arg-His-Asp-Ser-Gly-Tyr-Glu-Val-
His-His-Gln-X-X-Val-Phe-Phe-Ala-Glu-Asp-Val-Gly-Ser-Asn-Lys-
Gly-Ala-Ile-Ile-Gly-Leu-Met-Val-Gly-Gly-Val-Val,
 wherein X represents any amino acid residue.

19. A fusion protein comprising a modified protein according to Claim 17 or 18 and a detectable secretory protein.

20. A nucleic acid molecule which encodes a protein according to any one of Claims 11 to 19.

21. A test cell which expresses an APP modified protein according to any one of Claims 11, 13, 14, 17, and 18.

22. A test cell which expresses a fusion protein according to any one of Claims 12, 15, 16, and 19.

23. A method for examining whether or not a subject substance affects β-secretase activity, the method comprising culturing in the presence of the subject substance a test cell according to Claim 22, and measuring detectable secretory proteins which were cleaved by β-secretase and secreted outside the cell.

24. A method for examining whether or not a subject substance affects β-amyloid production, the method comprising culturing in the presence of the subject substance a test cell according to Claim 22, and measuring detectable secretory proteins which were cleaved by β-secretase and secreted outside the cell.

25. A method for screening a substance that is useful as an active ingredient for a therapeutic agent or prophylactic agent for Alzheimer's-type dementia, the screening method comprising culturing in the presence of a subject substance a test cell according to Claim 22, and measuring detectable secretory proteins which were cleaved by β-secretase and secreted outside the cell.

26. A method for examining the cytotoxicity of a subject substance according to any one of Claims 23 to 25, the method further comprising performing a cell viability test of the test cell cultured in the presence of the subject substance.
